# EUROPÄISCHE PATENTANMELDUNG

(11) **EP 1 903 054 A2**
(43) Veröffentlichungstag der Anmeldung: **26.03.2008**
(21) Anmeldenummer: 07023083.4
(22) Anmeldetag: 05.06.2002
(51) Int. Cl.: C07K 14/11, C07K 14/47, A61K 48/00, A61K 39/00, A61K 39/145, A61P 31/16, A61P 35/04

(54) **Stabilisierte mRNA mit erhöhtem G/C-Gehalt und optimierter Codon Usage für die Gentherapie**

(30) Priorität: 05.06.2001 DE 10127283
(62) Teilanmeldung aus: 05004938.6
(71) Anmelder: CureVac GmbH, 72076 Tübingen (DE)
(72) Erfinder: von der Mülbe, Florian, 72070 Tübingen (DE); Pascolo, Steve, Dr., 72072 Tübingen (DE); Hoerr, Ingmar, Dr., 72070 Tübingen (DE)
(74) Vertreter: Graf von Stosch, Andreas

(57) **Zusammenfassung**

Die vorliegende Erfindung betrifft eine pharmazeutische Zusammensetzung, enthaltend eine mRNA, die durch Sequenzmodifikationen im translatierten Bereich stabilisiert und für die Translation optimiert ist. Die erfindungsgemäße pharmazeutische Zusammensetzung eignet sich insbesondere als Impfstoff sowie als Therapeutikum zur Geweberegeneration. Des weiteren wird ein Verfahren zur Ermittlung von Sequenzmodifikationen, die der Stabilisierung und Translationsoptimierung von mRNA dierien, offenbart.

SEQUENCE LISTING

<110> CureVac GmbH

<120> Pharmazeutische Zusammensetzung, enthaltend eine
      stabilisierte und für die Translation in ihren
      codierenden Bereichen optimierte mRNA

<130> CU01P003WOEPT5

<150> PCT/EP02/06180
<151> 2002-06-05

<160> 13

<170> PatentIn Ver. 2.1

<210> 1
<211> 774
<212> DNA
<213> Influenza virus

<220>
<223> Influenza-Matrix: Wildtyp-Gen (zum Vergleich)

<220>
<223> Startcodon: atg (Nucleotide 11 bis 13), Stopcodon:
      tga (Nucleotide 767 bis 769 )

<400> 1
agatctaaag atgagtcttc taaccgaggt cgaaacgtac gttctctcta tcatcccgtc 60
aggccccctc aaagccgaga tcgcacagag acttgaagat gtctttgcag ggaagaacac 120
cgatcttgag gttctcatgg aatggctaaa gacaagacca atcctgtcac ctctgactaa 180
ggggatttta ggatttgtgt tcacgctcac cgtgcccagt gagcgaggac tgcagcgtag 240
acgctttgtc caaaatgccc ttaatgggaa cggggatcca aataacatgg acaaagcagt 300
taaactgtat aggaagctca agagggagat aacattccat ggggccaaag aaatctcact 360
cagttattct gctggtgcac ttgccagttg tatgggcctc atatacaaca ggatgggggc 420
tgtgaccact gaagtggcat ttggcctggt atgtgcaacc tgtgaacaga ttgctgactc 480
ccagcatcgg tctcataggc aaatggtgac aacaaccaac ccactaatca gacatgagaa 540
cagaatggtt ttagccagca ctacagctaa ggctatggag caaatggctg gatcgagtga 600
gcaagcagca gaggccatgg aggttgctag tcaggctagg caaatggtgc aagcgatgag 660
aaccattggg actcatccta gctccagtgc tggtctgaaa aatgatcttc ttgaaaattt 720
gcaggcctat cagaaacgaa tgggggtgca gatgcaacgg ttcaagtgaa ctag      774


<210> 2
<211> 252
<212> PRT
<213> Influenza virus

<400> 2
Met Ser Leu Leu Thr Glu Val Glu Thr Tyr Val Leu Ser Ile Ile Pro
  1               5                  10                  15

Ser Gly Pro Leu Lys Ala Glu Ile Ala Gln Arg Leu Glu Asp Val Phe
             20                  25                  30

Ala Gly Lys Asn Thr Asp Leu Glu Val Leu Met Glu Trp Leu Lys Thr
         35                  40                  45

Arg Pro Ile Leu Ser Pro Leu Thr Lys Gly Ile Leu Gly Phe Val Phe
     50                  55                  60

Thr Leu Thr Val Pro Ser Glu Arg Gly Leu Gln Arg Arg Arg Phe Val

65                  70                  75                  80

Gln Asn Ala Leu Asn Gly Asn Gly Asp Pro Asn Asn Met Asp Lys Ala
                85                  90                  95

Val Lys Leu Tyr Arg Lys Leu Lys Arg Glu Ile Thr Phe His Gly Ala
            100                 105                 110

Lys Glu Ile Ser Leu Ser Tyr Ser Ala Gly Ala Leu Ala Ser Cys Met
        115                 120                 125

Gly Leu Ile Tyr Asn Arg Met Gly Ala Val Thr Thr Glu Val Ala Phe
    130                 135                 140

Gly Leu Val Cys Ala Thr Cys Glu Gln Ile Ala Asp Ser Gln His Arg
145                 150                 155                 160

Ser His Arg Gln Met Val Thr Thr Thr Asn Pro Leu Ile Arg His Glu
                165                 170                 175

Asn Arg Met Val Leu Ala Ser Thr Thr Ala Lys Ala Met Glu Gln Met
            180                 185                 190

Ala Gly Ser Ser Glu Gln Ala Ala Glu Ala Met Glu Val Ala Ser Gln
        195                 200                 205

Ala Arg Gln Met Val Gln Ala Met Arg Thr Ile Gly Thr His Pro Ser
    210                 215                 220

Ser Ser Ala Gly Leu Lys Asn Asp Leu Leu Glu Asn Leu Gln Ala Tyr
225                 230                 235                 240

Gln Lys Arg Met Gly Val Gln Met Gln Arg Phe Lys
                245                 250


<210> 3
<211> 775
<212> DNA
<213> Artificial Sequence

<220>
<223> Description of Artificial Sequence:
      Influenza-Matrix: Gen mit erhöhtem G/C-Gehalt

<220>
<223> Startcodon: atg (Nucleotide 11 bis 13), Stopcodon:
      tga (Nucleotide 767 bis 769)

<400> 3
agatctaaag atgagcctgc tgaccgaggt ggagacctac gtgctgagca tcatccccag 60
cggccccctg aaggccgaga tcgcccagag gctggaggac gtgttcgccg gcaagaacac 120
cgacctggag gtgctgatgg agtggctgaa gaccaggccc atcctgagcc ccctgaccaa 180
gggcatcctg ggcttcgtgt tcaccctgac cgtgcccagc gagcgcggcc tgcagcgccg 240
ccgcttcgtg cagaacgccc tgaacggcaa cggcgacccc aacaacatgg acaaggccgt 300
gaagctgtac aggaagctga agagggagat caccttccac ggcgccaagg agatcagcct 360
gagctacagc gccggcgccc tggccagctg catgggcctg atctacaaca ggatgggcgc 420
cgtgaccacc gaggtggcct tcggcctggt gtgcgccacc tgcgagcaga tcgccgacag 480
ccagcaccgc agccacaggc agatggtgac caccaccaac cccctgatca ggcacgagaa 540
caggatggtg ctggccagca ccaccgccaa ggccatggag cagatggccg gcagcagcga 600
gcaggccgcc gaggccatgg aggtggccag ccaggccagg cagatggtgc aggccatgag 660
gaccatcggc acccacccca gcagcagcgc cggcctgaag aacgacctgc tggagaacct 720
gcaggcctac cagaagcgca tgggcgtgca gatgcagcgc ttcaagtgaa ctagt 775

<210> 4
<211> 844
<212> DNA
<213> Artificial Sequence

<220>
<223> Description of Artificial Sequence:
      Influenza-Matrix: Gen für sekretierte Form (mit
      N-terminaler Signalsequenz) mit erhöhtem
      G/C-Gehalt

<220>
<223> Startcodon: atg (Nucleotide 11 bis 13), Stopcodon:
      tga (Nucleotide 836 bis 838)

<400> 4
agatctaaag atggccgtca tggccccccg caccctggtg ctgctgctga gcggcgccct 60
ggccctgacc cagacctggg ctagcctgct gaccgaggtg gagacctacg tgctgagcat 120
catccccagc ggccccctga aggccgagat cgcccagagg ctggaggacg tgttcgccgg 180
caagaacacc gacctggagg tgctgatgga gtggctgaag accaggccca tcctgagccc 240
cctgaccaag ggcatcctgg gcttcgtgtt caccctgacc gtgcccagcg agcgcggcct 300
gcagcgccgc cgcttcgtgc agaacgccct gaacggcaac ggcgacccca acaacatgga 360
caaggccgtg aagctgtaca ggaagctgaa gagggagatc accttccacg gcgccaagga 420
gatcagcctg agctacagcg ccggcgccct ggccagctgc atgggcctga tctacaacag 480
gatgggcgcc gtgaccaccg aggtggcctt cggcctggtg tgcgccacct gcgagcagat 540
cgccgacagc cagcaccgca gccacaggca gatggtgacc accaccaacc ccctgatcag 600
gcacgagaac aggatggtgc tggccagcac caccgccaag gccatggagc agatggccgg 660
cagcagcgag caggccgccg aggccatgga ggtggccagc caggccaggc agatggtgca 720
ggccatgagg accatcggca cccaccccag cagcagcgcc ggcctgaaga acgacctgct 780
ggagaacctg caggcctacc agaagcgcat gggcgtgcag atgcagcgct tcaagtgaac 840
tagt 844


<210> 5
<211> 942
<212> RNA
<213> Artificial Sequence

<220>
<223> Description of Artificial Sequence:
      Influenza-Matrix: mRNA mit
      Stabilisierungssequenzen

<220>
<223> Die Stabilisierungssequenzen stammen aus den 5'-
      bzw. 3'-UTRs der ß-Globin-mRNA von Xenopus laevis

<220>
<223> Startcodon: aug (Nucleotide 56 bis 58), Stopcodon:
      uga (Nucleotide 812 bis 814)

<400> 5
gcuuguucuu uuugcagaag cucagaauaa acgcucaacu uuggcagauc uaaagaugag 60
ucuucuaacc gaggucgaaa cguacguucu cucuaucauc ccgucaggcc cccucaaagc 120
cgagaucgca cagagacuug aagaugucuu ugcagggaag aacaccgauc uugagguucu 180
cauggaaugg cuaaagacaa gaccaauccu gucaccucug acuaagggga uuuuaggauu 240
uguguucacg cucaccgugc ccagugagcg aggacugcag cguagacgcu uuguccaaaa 300
ugcccuuaau gggaacgggg auccaaauaa cauggacaaa gcaguuaaac uguauaggaa 360
gcucaagagg gagauaacau uccauggggc caaagaaauc ucacucaguu auucugcugg 420
ugcacuugcc aguuguaugg gccucauaua caacaggaug gggcugugaa ccacugaagu 480
ggcauuuggc cugguaugug caaccuguga acagauugcu gacucccagc aucggucuca 540
uaggcaaaug gugacaacaa ccaacccacu aaucagacau gagaacagaa ugguuuuagc 600
cagcacuaca gcuaaggcua uggagcaaau ggcuggaucg agugagcaag cagcagaggc 660

cauggagguu gcuagucagg cuaggcaaau ggugcaagcg augagaacca uugggacuca 720
uccuagcucc agugcugguc ugaaaaauga ucuucuugaa aauuugcagg ccuaucagaa 780
acgaaugggg gugcagaugc aacgguucaa gugaacuagu gacugacuag cccgcugggc 840
cucccaacgg gcccuccucc ccuccuugca ccaaaaaaaa aaaaaaaaaa aaaaaaaaaa 900
aaaaaaaaaa aaaaaaaaaa aaaaaaaaaa aaaaaaaaaa aa 942

<210> 6
<211> 942
<212> RNA
<213> Artificial Sequence

<220>
<223> Description of Artificial Sequence:
      Influenza-Matrix: mRNA mit erhöhtem G/C-Gehalt und
      Stabilisierungssequenzen

<220>
<223> Die Stabilisierungssequenzen stammen aus den 5'-
      bzw. 3'-UTRs der ß-Globin-mRNA von Xenopus laevis

<220>
<223> Startcodon: aug (Nucleotide 56 bis 58), Stopcodon:
      uga (Nucleotide 812 bis 814)

<400> 6
gcuuguucuu uuugcagaag cucagaauaa acgcucaacu uuggcagauc uaaagaugag 60
ccugcugacc gagguggaga ccuacgugcu gagcaucauc cccagcggcc cccugaaggc 120
cgagaucgcc cagaggcugg aggacguguu cgccggcaag aacaccgacc uggaggugcu 180
gauggagugg cugaagacca ggcccauccu gagcccccug accaagggca uccugggcuu 240
cguguucacc cugaccgugc ccagcgagcg cggccugcag cgccgccgcu ucgugcagaa 300
cgcccugaac ggcaacggcg accccaacaa cauggacaag gccgugaagc uguacaggaa 360
gcugaagagg gagaucaccu uccacggcgc caaggagauc agccugagcu acagcgccgg 420
cgcccuggcc agcugcaugg gccugaucua caacaggaug ggcgccguga ccaccgaggu 480
ggccuucggc cuggugugcg ccaccugcga gcagaucgcc gacagccagc accgcagcca 540
caggcagaug gugaccacca ccaacccccu gaucaggcac gagaacagga uggugcuggc 600
cagcaccacc gccaaggcca uggagcagau ggccggcagc agcgagcagg ccgccgaggc 660
cauggaggug gccagccagg ccaggcagau ggugcaggcc augaggacca ucggcaccca 720
ccccagcagc agcgccggcc ugaagaacga ccugcuggag aaccugcagg ccuaccagaa 780
gcgcaugggc gugcagaugc agcgcuucaa gugaacuagu gacugacuag cccgcugggc 840
cucccaacgg gcccuccucc ccuccuugca ccaaaaaaaa aaaaaaaaaa aaaaaaaaaa 900
aaaaaaaaaa aaaaaaaaaa aaaaaaaaaa aaaaaaaaaa aa 942

<210> 7
<211> 1011
<212> RNA
<213> Artificial Sequence

<220>
<223> Description of Artificial Sequence:
      Influenza-Matrix: für sekretierte Form codierende
      mRNA mit erhöhtem G/C-Gehalt und
      Stabilisierungssequenzen

<220>
<223> Startcodon: aug (Nucleotide 56 bis 58), Stopcodon:
      uga (Nucleotide 881 bis 883)

<400> 7
gcuuguucuu uuugcagaag cucagaauaa acgcucaacu uuggcagauc uaaagauggc 60
cgucauggcc ccccgcaccc uggugcugcu gcugagcggc gcccuggccc ugacccagac 120
cugggccagc cugcugaccg agguggagac cuacgugcug agcaucaucc ccagcggccc 180
ccugaaggcc gagaucgccc agaggcugga ggacguguuc gccggcaaga acaccgaccu 240

ggaggugcug auggaguggc ugaagaccag gcccauccug agcccccuga ccaagggcau 300
ccugggcuuc guguucaccc ugaccgugcc cagcgagcgc ggccugcagc gccgccgcuu 360
cgugcagaac gcccugaacg gcaacggcga ccccaacaac auggacaagg ccgugaagcu 420
guacaggaag cugaagaggg agaucaccuu ccacggcgcc aaggagauca gccugagcua 480
cagcgccggc gcccuggcca gcugcauggg ccugaucuac aacaggaugg gcgccgugac 540
caccgaggug gccuucggcc uggugugcgc caccugcgag cagaucgccg acagccagca 600
ccgcagccac aggcagaugg ugaccaccac caacccccug aucaggcacg agaacaggau 660
ggugcuggcc agcaccaccg ccaaggccau ggagcagaug gccggcagca gcgagcaggc 720
cgccgaggcc auggaggugg ccagccaggc caggcagaug gugcaggcca ugaggaccau 780
cggcacccac cccagcagca gcgccggccu gaagaacgac cugcuggaga accugcaggc 840
cuaccagaag cgcaugggcg ugcagaugca gcgcuucaag ugaacuagug acugacuagc 900
ccgcugggcc ucccaacggg cccuccuccc cuccuugcac caaaaaaaaa aaaaaaaaaa 960
aaaaaaaaaa aaaaaaaaaa aaaaaaaaaa aaaaaaaaaa aaaaaaaaaa a 1011

<210> 8
<211> 940
<212> DNA
<213> Homo sapiens

<220>
<223> MAGE1: Wildtyp-Gen (zum Vergleich)

<220>
<223> Startcodon: atg (Nucleotide 5 bis 7), Stopcodon:
tga (Nucleotide 932 bis 934)

<400> 8
catcatgtct cttgagcaga ggagtctgca ctgcaagcct gaggaagccc ttgaggccca 60
acaagaggcc ctgggcctgg tgtgtgtgca ggctgccacc tcctcctcct ctcctctggt 120
cctgggcacc ctggaggagg tgcccactgc tgggtcaaca gatcctcccc agagtcctca 180
gggagcctcc gcctttccca ctaccatcaa cttcactcga cagaggcaac ccagtgaggg 240
ttccagcagc cgtgaagagg aggggccaag cacctcttgt atcctggagt ccttgttccg 300
agcagtaatc actaagaagg tggctgattt ggttggtttt ctgctcctca aatatcgagc 360
cagggagcca gtcacaaagg cagaaatgct ggagagtgtc atcaaaaatt acaagcactg 420
ttttcctgag atcttcggca aagcctctga gtccttgcag ctggtctttg gcattgacgt 480
gaaggaagca gaccccaccg gccactccta tgtccttgtc acctgcctag gtctctccta 540
tgatggcctg ctgggtgata atcagatcat gcccaagaca ggcttcctga taattgtcct 600
ggtcatgatt gcaatggagg gcggccatgc tcctgaggag gaaatctggg aggagctgag 660
tgtgatggag gtgtatgatg ggagggagca cagtgcctat ggggagccca ggaagctgct 720
cacccaagat ttggtgcagg aaaagtacct ggagtaccgg caggtgccgg acagtgatcc 780
cgcacgctat gagttcctgt ggggtccaag ggccctcgct gaaaccagct atgtgaaagt 840
ccttgagtat gtgatcaagg tcagtgcaag agttcgcttt ttcttcccat ccctgcgtga 900
agcagctttg agagaggagg aagagggagt ctgagcatga 940

<210> 9
<211> 308
<212> PRT
<213> Homo sapiens

<220>
<223> Tumorantigen MAGE1: Proteinsequenz

<400> 9
Ser Leu Glu Gln Arg Ser Leu His Cys Lys Pro Glu Glu Ala Leu Glu
1               5                   10                  15

Ala Gln Gln Glu Ala Leu Gly Leu Val Cys Val Gln Ala Ala Thr Ser
            20                  25                  30

Ser Ser Ser Pro Leu Val Leu Gly Thr Leu Glu Glu Val Pro Thr Ala
        35                  40                  45

Gly Ser Thr Asp Pro Pro Gln Ser Pro Gln Gly Ala Ser Ala Phe Pro
    50                  55                  60

Thr Thr Ile Asn Phe Thr Arg Gln Arg Gln Pro Ser Glu Gly Ser Ser
65                  70                  75                  80

Ser Arg Glu Glu Glu Gly Pro Ser Thr Ser Cys Ile Leu Glu Ser Leu
                85                  90                  95

Phe Arg Ala Val Ile Thr Lys Lys Val Ala Asp Leu Val Gly Phe Leu
            100                 105                 110

Leu Leu Lys Tyr Arg Ala Arg Glu Pro Val Thr Lys Ala Glu Met Leu
        115                 120                 125

Glu Ser Val Ile Lys Asn Tyr Lys His Cys Phe Pro Glu Ile Phe Gly
    130                 135                 140

Lys Ala Ser Glu Ser Leu Gln Leu Val Phe Gly Ile Asp Val Lys Glu
145                 150                 155                 160

Ala Asp Pro Thr Gly His Ser Tyr Val Leu Val Thr Cys Leu Gly Leu
                165                 170                 175

Ser Tyr Asp Gly Leu Leu Gly Asp Asn Gln Ile Met Pro Lys Thr Gly
            180                 185                 190

Phe Leu Ile Ile Val Leu Val Met Ile Ala Met Glu Gly Gly His Ala
        195                 200                 205

Pro Glu Glu Glu Ile Trp Glu Glu Leu Ser Val Met Glu Val Tyr Asp
    210                 215                 220

Gly Arg Glu His Ser Ala Tyr Gly Glu Pro Arg Lys Leu Leu Thr Gln
225                 230                 235                 240

Asp Leu Val Gln Glu Lys Tyr Leu Glu Tyr Arg Gln Val Pro Asp Ser
                245                 250                 255

Asp Pro Ala Arg Tyr Glu Phe Leu Trp Gly Pro Arg Ala Leu Ala Glu
            260                 265                 270

Thr Ser Tyr Val Lys Val Leu Glu Tyr Val Ile Lys Val Ser Ala Arg
        275                 280                 285

Val Arg Phe Phe Phe Pro Ser Leu Arg Glu Ala Ala Leu Arg Glu Glu
    290                 295                 300

Glu Glu Gly Val
305


<210> 10
<211> 939
<212> RNA
<213> Artificial Sequence

<220>
<223> Description of Artificial Sequence: MAGE1: mRNA
      mit erhöhtem G/C-Gehalt

<220>
<223> Startcodon: aug (Nucleotide 1 bis 3), Stopcodon:
      uga (Nucleotide 937 bis 939)

<400> 10
augagccugg agcagcgcag ccugcacugc aagccggagg aggcgcugga ggcgcagcag 60
gaggcgcugg gccuggucug cguccaggcg gcgacgagca gcagcagccc gcugguccug 120
ggcacgcugg aggagguccc gacggcgggc agcacggacc cgccgcagag cccgcagggc 180
gcgagcgcgu ucccgacgac gaucaacuuc acgcgccagc gccagccgag cgagggcagc 240
agcagccgcg aggaggaggg cccgagcacg agcugcaucc uggagagccu guuccgcgcg 300
gucaucacga agaaggucgc ggaccugguc ggcuuccugc ugcugaagua ccgcgcgcgc 360
gagccgguca cgaaggcgga gaugcuggag agcgucauca agaacuacaa gcacugcuuc 420
ccggagaucu ucggcaaggc gagcgagagc cugcagcugg ucuucggcau cgacgucaag 480
gaggcggacc cgacgggcca cagcuacguc cuggucacgu gccugggccu gagcuacgac 540
ggccugcugg gcgacaacca gaucaugccg aagacgggcu uccugaucau cguccugguc 600
augaucgcga uggagggcgg ccacgcgccg gaggaggaga ucugggagga gcugagcguc 660
auggaggucu acgacggccg cgagcacagc gcguacggcg agccgcgcaa gcugcugacg 720
caggaccugg uccaggagaa guaccuggag uaccgccagg ucccggacag cgacccggcg 780
cgcuacgagu uccugugggg cccgcgcgcg cuggcggaga cgagcuacgu caagguccug 840
gaguacguca ucaaggucag cgcgcgcguc cgcuucuucu ucccgagccu gcgcgaggcg 900
gcgcugcgcg aggaggagga gggcgucuga gcgugauga 939

<210> 11
<211> 939
<212> RNA
<213> Artificial Sequence

<220>
<223> Description of Artificial Sequence: MAGE1: mRNA
      mit alternativer Codonverwendung

<220>
<223> Startcodon: aug (Nucleotide 1 bis 3), Stopcodon:
      uga (Nucleotide 937 bis 939)

<400> 11
augagccugg agcagcgcag ccugcacugc aagcccgagg aggcccugga ggcccagcag 60
gaggcccugg gccuggugug cgugcaggcc gccaccagca gcagcagccc ccuggugcug 120
ggcacccugg aggaggugcc caccgccggc agcaccgacc ccccccagag cccccagggc 180
gccagcgccu uccccaccac caucaacuuc acccgccagc gccagcccag cgagggcagc 240
agcagccgcg aggaggaggg ccccagcacc agcugcaucc uggagagccu guuccgcgcc 300
gugaucacca agaagguggc cgaccuggug ggcuuccugc ugcugaagua ccgcgcccgc 360
gagcccguga ccaaggccga gaugcuggag agcgugauca agaacuacaa gcacugcuuc 420
cccgagaucu ucggcaaggc cagcgagagc cugcagcugg uguucggcau cgacgugaag 480
gaggccgacc ccaccggcca cagcuacgug cuggugaccu gccugggccu gagcuacgac 540
ggccugcugg gcgacaacca gaucaugccc aagaccggcu uccugaucau cgugcuggug 600
augaucgcca uggagggcgg ccacgccccc gaggaggaga ucugggagga gcugagcgug 660
auggaggugu acgacggccg cgagcacagc gccuacggcg agccccgcaa gcugcugacc 720
caggaccugg ugcaggagaa guaccuggag uaccgccagg ugcccgacag cgaccccgcc 780
cgcuacgagu uccugugggg cccccgcgcc cuggccgaga ccagcuacgu gaaggugcug 840
gaguacguga ucaaggugag cgcccgcgug cgcuucuucu uccccagccu gcgcgaggcc 900
gcccugcgcg aggaggagga gggcguguga gccugauga 939

<210> 12
<211> 7
<212> RNA
<213> Artificial Sequence

<220>
<223> Description of Artificial Sequence: Ein für eine
      Endonuklease erkennbares Sequenzmotiv, das im
      3'UTR-Segment des für den Transferin-Rezeptor
      codierenden Gens enthalten ist (S. 10 der
      Beschreibung).

<400> 12
gaacaag                                                                  7


<210> 13
<211> 13
<212> RNA
<213> Artificial Sequence

<220>
<223> Description of Artificial Sequence: Kozak-Sequenz,
      Ribosomen-Bindungsstelle (S. 12 der Beschreibung)

<400> 13
gccgccacca ugg                                                          13

## Beschreibung

Die vorliegende Erfindung betrifft eine pharmazeutische Zusammensetzung, enthaltend eine mRNA, die durch Sequenzmodifikationen im translatierten Bereich stabilisiert und für die Translation optimiert ist. Die erfindungsgemäße pharmazeutische Zusammensetzung eignet sich insbesondere als Impfstoff sowie als Therapeutikum zur Geweberegeneration. Des weiteren wird ein Verfahren zur Ermittlung von Sequenzmodifikationen, die der Stabilisierung und Translationsoptimierung von mRNA dienen, offenbart.

Die Gentherapie und die genetische Vakzinierung sind molekularmedizinische Verfahren, deren Anwendung in der Therapie und Prävention von Erkrankungen erhebliche Auswirkungen auf die medizinische Praxis haben wird. Beide Verfahren beruhen auf der Einbringung von Nukleinsäuren in Zellen bzw. in Gewebe des Patienten sowie auf der anschließenden Verarbeitung der durch die eingebrachten Nukleinsäuren codierten Information, d.h. der Expression der erwünschten Polypeptide.

Die übliche Vorgehensweise bisheriger Verfahren der Gentherapie und der genetischen Vakzinierung ist die Verwendung von DNA, um die benötigte genetische Information in die Zelle einzuschleusen. In diesem Zusammenhang sind verschiedene Verfahren zur Einbringung von DNA in Zellen, wie bspw. Calciumphosphat-Transfektion, Polypren-Transfektion, Protoplasten-Fusion, Elektroporation, Mikroinjektion und Lipofektion, entwickelt worden, wobei sich insbesondere die Lipofektion als geeignetes Verfahren herausgestellt hat.

Ein weiteres Verfahren, das insbesondere bei genetischen Vakzinierungsverfahren vorgeschlagen wurde, ist die Verwendung von DNA-Viren als DNA-Vehikel. Derartige Viren haben den Vorteil, daß aufgrund ihrer infektiösen Eigenschaften eine sehr hohe Transfektionsrate zu erzielen ist. Die verwendeten Viren werden genetisch verändert, so daß in der transfizierten Zelle keine funktionsfähigen infektiösen Partikel gebildet werden. Trotz dieser Vorsichtsmaßnahme kann jedoch aufgrund möglicher Rekombinationsereignisse ein gewisses Risiko der unkontrollierten Ausbreitung der eingebrachten gentherapeutisch wirksamen sowie viralen Gene nicht ausgeschlossen werden.

Üblicherweise wird die in die Zelle eingebrachte DNA in gewissem Ausmaß in das Genom der transfizierten Zelle integriert. Einerseits kann dieses Phänomen einen erwünschten Effekt ausüben, da hierdurch eine langandauernde Wirkung der eingebrachten DNA erzielt werden kann. Andererseits bringt die Integration in das Genom ein wesentliches Risiko der Gentherapie mit sich. So kann es bspw. zu einer Insertion der eingebrachten DNA in ein intaktes Gen kommen, was eine Mutation darstellt, welche die Funktion des endogenen Gens behindert oder gar vollkommen ausschaltet. Durch solche Integrationsereignisse können einerseits für die Zelle lebenswichtige Enzymsysteme ausgeschaltet werden, andererseits besteht auch die Gefahr einer Transformation der so veränderten Zelle in einen entarteten Zustand, falls durch die Integration der Fremd-DNA ein für die Regulation des Zellwachstums entscheidendes Gen verändert wird. Daher kann bei der Verwendung von DNA-Viren als Gentherapeutika und Vakzine ein Risiko der Krebsbildung nicht ausgeschlossen werden. In diesem Zusammenhang ist auch zu beachten, daß zur wirksamen Expression der in die Zelle eingebrachten Gene die entsprechenden DNA-Vehikel einen starken Promotor, bspw. den viralen CMV-Promotor, enthalten. Die Integration derartiger Promotoren in das Genom der behandelten Zelle kann zu unerwünschten Veränderungen der Regulierung der Genexpression in der Zelle fuhren.

Ein weiterer Nachteil der Verwendung von DNA als Gentherapeutika und Vakzine ist die Induktion pathogener Anti-DNA-Antikörper im Patienten unter Hervorrufung einer möglicherweise tödlichen Immunantwort.

Im Gegensatz zu DNA ist der Einsatz von RNA als Gentherapeutikum oder Vakzin als wesentlich sicherer einzustufen. Insbesondere bringt RNA nicht die Gefahr mit sich, stabil in das Genom der transfizierten Zelle integriert zu werden. Des weiteren sind keine viralen Sequenzen, wie Promotoren, zur wirksamen Transkription, erforderlich. Darüber hinaus wird RNA wesentlich einfacher *in vivo* abgebaut. Wohl aufgrund der gegenüber DNA relativ kurzen Halbwertszeit von RNA im Blutkreislauf sind bisher keine anti-RNA-Antikörper nachgewiesen worden. Daher kann RNA für molekularmedizinische Therapieverfahren als Molekül der Wahl angesehen werden.

Allerdings bedürfen auf RNA-Expressionssystemen beruhende medizinische Verfahren vor einer breiteren Anwendung noch der Lösung einiger grundsätzlicher Probleme. Eines der Probleme bei der Verwendung von RNA ist der sichere, Zell- bzw. Gewebespezifische effiziente Transfer der Nukleinsäure. Da sich RNA in Lösung normalerweise als sehr instabil erweist, konnte durch die herkömmlichen Verfahren, die bei DNA verwendet werden, RNA bisher nicht oder nur sehr ineffizient als Therapeutikum bzw. Impfstoff verwendet werden.

Für die Instabilität sind RNA-abbauende Enzyme, sog. RNAasen (Ribonucleasen), verantwortlich. Selbst kleinste Verunreinigungen von Ribonucleasen reichen aus, um RNA in Lösung vollständig abzubauen. Der natürliche Abbau von mRNA im Cytoplasma von Zellen ist sehr fein reguliert. Diesbezüglich sind mehrere Mechanismen bekannt. So ist für eine funktionale mRNA die endständige Struktur von entscheidender Bedeutung. Am 5'-Ende befindet sich die sogenannte "Cap-Struktur" (ein modifiziertes Guanosin-Nucleotid) und am 3'-Ende eine Abfolge von bis zu 200 Adenosin-Nucleotiden (der sog. Poly-A-Schwanz). Über diese Strukturen wird die RNA als mRNA erkannt und der Abbau reguliert. Darüber hinaus gibt es weitere Prozesse, die RNA stabilisieren bzw. destabilisieren. Viele diese Prozesse sind noch unbekannt, oftmals scheint jedoch eine Wechselwirkung zwischen der RNA und Proteinen dafür maßgeblich zu sein. Bspw. wurde kürzlich ein "mRNA-Surveillance-System" beschrieben (Hellerin und Parker, Annu. Rev. Genet. 1999, 33: 229 bis 260), bei dem durch bestimmte Feedback-Protein-Wechselwirkungen im Cytosol unvollständige oder Nonsense-mRNA erkannt und dem Abbau zugänglich gemacht wird, wobei ein Hauptteil dieser Prozesse durch Exonucleasen vollzogen wird.

Im Stand der Technik sind einige Maßnahmen vorgeschlagen worden, um die Stabilität von RNA zu erhöhen und dadurch ihren Einsatz als Gentherapeutikum bzw. RNA-Vakzine zu ermöglichen.

In EP-A-1083232 wird zur Lösung des vorstehend genannten Problems der Instabilität von RNA *ex vivo* ein Verfahren zur Einbringung von RNA, insbesondere mRNA, in Zellen und Organismen vorgeschlagen, bei welchem die RNA in Form eines Komplexes mit einem kationischen Peptid oder Protein vorliegt.

WO 99/14346 beschreibt weitere Verfahren zur Stabilisierung von mRNA. Insbesondere werden Modifizierungen der mRNA vorgeschlagen, welche die mRNA-Spezies gegenüber dem Abbau von RNasen stabilisieren. Derartige Modifikationen betreffen einerseits die Stabilisierung durch Sequenzmodifikationen, insbesondere Verminderung des C-und/oder U-Gehalts durch Baseneliminierung oder Basensubstitution. Andererseits werden chemische Modifikationen, insbesondere die Verwendung von Nucleotidanaloga, sowie 5'- und 3'- Blockierungsgruppen, eine erhöhte Länge des Poly-A-Schwanzes sowie die Komplexierung der mRNA mit stabilisierenden Mitteln und Kombinationen der genannten Maßnahmen vorgeschlagen.

In den US-Patenten US 5,580,859 und US 6,214,804 werden unter anderem im Rahmen der "transienten Gentherapie" (TGT) mRNA-Vakzine und -Therapeutika offenbart. Es werden verschiedene Maßnahmen zur Erhöhung der Translationseffizienz und der mRNA-Stabilität beschrieben, die sich vor allem auf die nicht-translatierten Sequenzbereiche beziehen.

Bieler und Wagner (in: Schleef (Hrsg.), Plasmids for Therapy and Vaccination, Kapitel 9, Seiten 147 bis 168, Wiley-VCH, Weinheim, 2001) berichten von der Verwendung synthetischer Gene im Zusammenhang mit gentherapeutischen Methoden unter Verwendung von DNA-Vakzinen und lentiveralen Vektoren. Es wird die Konstruktion eines synthetischen, von HIV-1 abgeleiteten *gag*-Gens beschrieben, bei welchem die Codons gegenüber der Wildtyp-Sequenz derart modifiziert wurden (alternative Codonverwendung, engl. "codon usage"), daß sie der Verwendung von Codons entsprach, die in hoch exprimierten Säugergenen zu finden ist. Dadurch wurde insbesondere der A/T-Gehalt gegenüber der Wildtyp-Sequenz vermindert. Die Autoren stellen insbesondere eine erhöhte Expressionsrate des synthetischen gag-Gens in transfizierten Zellen fest. Des weiteren wurde in Mäusen eine erhöhte Antikörperbildung gegen das *gag*-Protein bei mit dem synthetischen DNA-Konstrukt immunisierten Mäusen und auch eine verstärkte Cytokinfreisetzung *in vitro* bei transfizierten Milzzellen von Mäusen beobachtet. Schließlich konnte eine Induzierung einer cytotoxischen Immunantwort in mit dem *gag*-Expressionsplasmid immunisierten Mäusen festgestellt werden. Die Autoren dieses Artikels führen die verbesserten Eigenschaften ihres DNA-Vakzins im wesentlichen auf einen durch die optimierte Codonverwendung hervorgerufene Veränderung des nucleo-cytoplasmatischen Transports der vom DNA-Vakzin exprimierten mRNA zurück. Im Gegensatz dazu halten die Autoren die Auswirkung der veränderten Codonverwendung auf die Translationseffizienz für gering.

Der vorliegenden Erfindung liegt daher die Aufgabe zugrunde, ein neues System zur Gentherapie und genetischen Vakzinierung bereitzustellen, das die mit den Eigenschaften von DNA-Therapeutika und -Vakzinen verbundenen Nachteile überwindet und die Wirksamkeit von auf RNA-Spezies basierenden Therapeutika erhöht.

Diese Aufgabe wird durch die in den Ansprüchen gekennzeichneten Ausführungsformen der vorliegenden Erfindung gelöst.

Insbesondere wird eine modifizierte mRNA sowie eine mindestens eine derartige modifizierte mRNA in Verbindung mit einem pharmazeutisch verträglichen Träger und/oder Vehikel enthaltende pharmazeutische Zusammensetzung bereitgestellt, wobei die modifizierte mRNA für mindestens ein biologisch wirksames oder antigenes Peptid oder Polypeptid codiert, wobei die Sequenz der mRNA, insbesondere im für das mindestens eine Peptid oder Polypeptid codierenden Bereich, gegenüber der Wildtyp-mRNA folgende Modifikationen aufweist, die entweder alternativ oder in Kombination vorliegen können.

Zum einen ist der G/C-Gehalt des für das Peptid oder Polypeptid codierenden Bereichs der modifizierten mRNA größer als der G/C-Gehalt des codierenden Bereichs der für das Peptid oder Polypeptid codierenden Wildtyp-mRNA, wobei die codierte Aminosäuresequenz gegenüber dem Wildtyp unverändert ist.

Diese Modifikation beruht auf der Tatsache, daß für eine effiziente Translation einer mRNA die Sequenzabfolge des zu translatierenden Bereichs der mRNA wesentlich ist. Dabei spielt die Zusammensetzung und die Abfolge der verschiedenen Nucleotide eine große Rolle. Insbesondere sind Sequenzen mit erhöhtem G(Guanosin)/C(Cytosin)-Gehalt stabiler als Sequenzen mit einem erhöhten A(Adenosin)/U(Uracil)-Gehalt. Daher werden erfindungsgemäß unter Beibehaltung der translatierten Aminosäureabfolge die Codons gegenüber der Wildtyp-mRNA derart variiert, daß sie vermehrt G/C-Nucleotide beinhalten. Da mehrere Codons für ein und dieselbe Aminosäure codieren (Degeneration des genetischen Codes), können die für die Stabilität günstigsten Codons ermittelt werden (alternative Codonverwendung, engl. "codon usage").

In Abhängigkeit von der durch die modifizierte mRNA zu codierenden Aminosäure sind unterschiedliche Möglichkeiten zur Modifikation der mRNA-Sequenz gegenüber der Wildtyp-Sequenz möglich. Im Fall von Aminosäuren, die durch Codons codiert werden, die ausschließlich G- oder C- Nucleotide enthalten, ist keine Modifikation des Codons erforderlich. So erfordern die Codons für Pro (CCC oder CCG), Arg (CGC oder CGG), Ala (GCC oder GCG) und Gly (GGC oder GGG) keine Veränderung, da kein A oder U vorhanden ist

In folgenden Fällen werden die Codons, welche A-und/oder U-Nucleotide enthalten durch Substituieren anderer Codons, welche die gleichen Aminosäuren codieren, jedoch kein A und/oder U enthalten, verändert. Beispiele sind:
Die Codons für Pro können von CCU oder CCA zu CCC oder CCG verändert werden;
die Codons für Arg können von CGU oder CGA oder AGA oder AGG zu CGC oder CGG verändert werden;
die Codons für Ala können von GCU oder GCA zu GCC oder GCG verändert werden;
die Codons für Gly können von GGU oder GGA zu GGC oder GGG verändert werden.

In anderen Fällen können A bzw. U-Nucleotide zwar nicht aus den Codons eliminiert werden, es ist jedoch möglich, den A- und U-Gehalt durch Verwendung von Codons zu vermindern, die weniger A- und/oder U-Nucleotide enthalten. Zum Beispiel:
Die Codons für Phe können von UUU zu UUC verändert werden;
die Codons für Leu können von UUA, CUU oder CUA zu CUC oder CUG verändert werden;
die Codons für Ser können von UCU oder UCA oder AGU zu UCC, UCG oder AGC verändert werden;
das Codon für Tyr kann von UAU zu UAC verändert werden;
das Stop-Codon UAA kann zu UAG oder UGA verändert werden;
das Codon für Cys kann von UGU zu UGC verändert werden;
das Codon His kann von CAU zu CAC verändert werden;
das Codon für Gln kann von CAA zu CAG verändert werden;
die Codons für IIe können von AUU oder AUA zu AUC verändert werden;
die Codons für Thr können von ACU oder ACA zu ACC oder ACG verändert werden;
das Codon für Asn kann von AAU zu AAC verändert werden;
das Codon für Lys kann von AAA zu AAG verändert werden;
die Codons für Val können von GUU oder GUA zu GUC oder GUG verändert werden;
das Codon für Asp kann von GAU zu GAC verändert werden;
das Codon für Glu kann von GAA zu GAG verändert werden.

Im Falle der Codons für Met (AUG) und Trp (UGG) besteht hingegen keine Möglichkeit der Sequenzmodifikation.

Die vorstehend aufgeführten Substitutionen können selbstverständlich einzeln aber auch in allen möglichen Kombinationen zur Erhöhung des G/C-Gehalts der modifizierten mRNA gegenüber der ursprünglichen Sequenz verwendet werden. So können beispielsweise alle in der ursprünglichen (Wildtyp-) Sequenz auftretenden Codons für Thr zu ACC (oder ACG) verändert werden. Vorzugsweise werden jedoch Kombinationen der vorstehenden Substitutionsmöglichkeiten genutzt, z.B.:
Substitution aller in der ursprünglichen Sequenz für Thr codierenden Codons zu ACC (oder ACG) und Substitution aller ursprünglich für Ser codierenden Codons zu UCC (oder UCG oder AGC);
Substitution aller in der ursprünglichen Sequenz für Ile codierenden Codons zu AUC und
Substitution aller ursprünglich für Lys codierenden Codons zu AAG und Substitution aller ursprünglich für Tyr codierenden Codons zu UAC;
Substitution aller in der ursprünglichen Sequenz für Val codierenden Codons zu GUC (oder GUG) und Substitution aller ursprünglich für Glu codierenden Codons zu GAG und
Substitution aller ursprünglich für Ala codierenden Codons zu GCC (oder GCG) und Substitution aller ursprünglich für Arg codierenden Codons zu CGC (oder CGG);
Substitution aller in der ursprünglichen Sequenz für Val codierenden Codons zu GUC (oder GUG) und Substitution aller ursprünglich für Glu codierenden Codons zu GAG und
Substitution aller ursprünglich für Ala codierenden Codons zu GCC (oder GCG) und Substitution aller ursprünglich für Gly codierenden Codons zu GGC (oder GGG) und Substituion aller ursprünglich für Asn codierenden Codons zu AAC;
Substitution aller in der ursprünglichen Sequenz für Val codierenden Codons zu GUC (oder GUG) und Substitution aller usprünglich für Phe codierenden Codons zu UUC und
Substitution aller ursprünglich für Cys codierenden Codons zu UGC und Substitution aller ursprünglich für Leu codierenden Codons zu CUG (oder CUC) und Substitution aller ursprünglich für Gln codierenden Codons zu CAG und Substitution aller ursprünglich für
Pro codierenden Codons zu CCC (oder CCG);
usw.

Vorzugsweise wird der G/C-Gehalt des für das Peptid bzw. Polypeptid codierenden Bereichs der modifizierten mRNA um mindestens 7%-Punkte, mehr bevorzugt um mindestens 15%-Punkte, besonders bevorzugt um mindestens 20%-Punkte gegenüber dem G/C-Gehalt des codierten Bereichs der für das Polypeptid codierenden Wildtyp-mRNA erhöht.

Besonders bevorzugt ist es in diesem Zusammenhang, den G/C-Gehalt der modifizierten mRNA, insbesondere im für das mindestens eine Peptid bzw. Polypeptid codierenden Bereich, im Vergleich zur Wildtyp-Sequenz maximal zu erhöhen.

Die weitere erfindungsgemäße Modifikation der in der vorliegenden Erfindung gekennzeichneten pharmazeutischen Zusammensetzung enthaltenen mRNA beruht auf der Erkenntnis, daß die Translationseffizienz auch durch eine unterschiedliche Häufigkeit im Vorkommen von tRNAs in Zellen bestimmt wird. Sind daher in einer RNA-Sequenz vermehrt sogenannte "seltene" Codons vorhanden, so wird die entsprechende mRNA deutlich schlechter translatiert als in dem Fall, wenn für relativ "häufige" tRNAs codierende Codons vorhanden sind.

Somit wird erfindungsgemäß in der modifizierten mRNA (welche in der pharmazeutischen Zusammensetzung enthalten ist), der für das Peptid bzw. Polypeptid codierende Bereich gegenüber dem entsprechenden Bereich der Wildtyp-mRNA derart verändert, daß mindestens ein Codon der Wildtyp-Sequenz, das für eine in der Zelle relativ seltene tRNA codiert, gegen ein Codon ausgetauscht, das für eine in der Zelle relativ häufige tRNA codiert, welche die gleiche Aminosäure trägt wie die relativ seltene tRNA.

Durch diese Modifikation werden die RNA-Sequenzen derart modifiziert, daß Codons eingefugt werden, für die häufig vorkommende tRNAs zur Verfügung stehen.

Welche tRNAs relativ häufig in der Zelle vorkommen und welche demgegenüber relativ selten sind, ist einem Fachmann bekannt; vgl. bspw. Akashi, Curr. Opin. Genet. Dev. 2001,11(6): 660-666.

Durch diese Modifikation können erfindungsgemäß alle Codons der Wildtyp-Sequenz, die für eine in der Zelle relativ seltene tRNA codieren, jeweils gegen ein Codon ausgetauscht werden, das für eine in der Zelle relativ häufige tRNA codiert, welche jeweils die gleiche Aminosäure trägt wie die relativ seltene tRNA.

Erfindungsgemäß besonders bevorzugt ist es, den erfindungsgemäß in der modifizierten mRNA erhöhten, insbesondere maximalen, sequenziellen G/C-Anteil mit den "häufigen" Codons zu verknüpfen, ohne die Aminosäuresequenz des durch den codierenden Bereich der mRNA codierten Peptids bzw. Polypeptids (ein oder mehrere) zu verändern. Diese bevorzugte Ausführungsform stellt eine besonders effizient translatierte und stabilisierte mRNA bspw. für die erfindungsgemäße pharmazeutische Zusammensetzung bereit.

In den Sequenzen eukaryotischer mRNAs gibt es destabilisierende Sequenzelemente (DSE), an welche Signalproteine binden und den enzymatischen Abbau der mRNA *in vivo* regulieren. Daher werden zur weiteren Stabilisierung der in der erfindungsgemäßen pharmazeutischen Zusammensetzung enthaltenen modifizierten mRNA gegebenenfalls im für das mindestens eine Peptid bzw. Polypeptid codierenden Bereich ein oder mehrere Veränderungen gegenüber dem entsprechenden Bereich der Wildtyp-mRNA vorgenommen, so daß keine destabilisierenden Sequenzelemente enthalten sind. Selbstverständlich ist es erfindungsgemäß ebenfalls bevorzugt, gegebenenfalls in den nicht-translatierten Bereichen (3'- und/oder 5'-UTR) vorhandene DSE aus der mRNA zu eliminieren.

Derartige destabilisierende Sequenzen sind bspw. AU-reiche Sequenzen ("AURES"), die in 3'-UTR-Abschnitten zahlreicher instabiler mRNA vorkommen (Caput et al., Proc. Natl. Acad. Sci. USA 1986, 83: 1670 bis 1674). Die in der erfindungsgemäßen pharmazeutischen Zusammensetzung enthaltenen RNA-Moleküle sind daher vorzugsweise derart gegenüber der Wildtyp-mRNA verändert, daß sie keine derartigen destabilisierenden Sequenzen aufweisen. Dies gilt auch für solche Sequenzmotive, die von möglichen Endonucleasen erkannt werden, bspw. die Sequenz GAACAAG, die im 3' UTR-Segment des für den Transferin-Rezeptor codierenden Gens enthalten ist (Binder et al., EMBO J. 1994, 13: 1969 bis 1980). Auch diese Sequenzmotive werden bevorzugt in der modifizierten mRNA der erfindungsgemäßen pharmazeutischen Zusammensetzung eliminiert.

Einem Fachmann sind verschiedene Verfahren geläufig, die zur Substitution von Codons in der erfindungsgemäßen modifizierten mRNA geeignet sind. Im Falle kürzerer codierender Bereiche (die für biologisch wirksame oder antigene Peptide codieren) kann bspw. die gesamte mRNA chemisch unter Verwendung von Standardtechniken synthetisiert werden.

Bevorzugt werden allerdings Basensubstitutionen unter Verwendung einer DNA-Matrize zur Herstellung der modifizierten mRNA mit Hilfe von Techniken der gängigen zielgerichteten Mutagenese eingeführt; Maniatis et al., Molecular Cloning: A Laboratory Manual, Cold Spring Harbor Laboratory Press, 3. Aufl., Cold Spring Harbor, NY, 2001.

Bei diesem Verfahren wird zur Herstellung der mRNA daher ein entsprechendes DNA-Molekül *in vitro* transkribiert. Diese DNA-Matrize besitzt einen geeigneten Promotor, bspw. einen T7 -oder SP6-Promotor, für die *in vitro* Transkription, dem die gewünschte Nucleotidsequenz für die herzustellende mRNA und ein Termisationssignal für die in *in vitro* Transkribtion folgen. Erfindungsgemäß wird das DNA-Molekül, das die Matrize des herzustellenden RNA-Konstrukts bildet, durch fermentative Vermehrung und anschließende Isolierung als Teil eines in Bakterien replizierbaren Plasmids hergestellt. Als für die vorliegende Erfindung geeignete Plasmide können bspw. die Plasmide p17Ts (GenBank-Zugriffsnummer U26404; Lai et al., Development 1995, 121: 2349 bis 2360), PGEM^{®}-Reihe, bspw. pGEM^{®}-1 (GenBank-Zugriffsnummer X65300; von Promega) und pSP64 (GenBank-Zugriffsnummer X65327) genannt werden; vgl. auch Mezei und Storts, Purification of PCR Products, in: Griffin und Griffin (Hrsg.), PCR Technology: Current Innovation, CRC Press, Boca Raton, FL, 2001.

Es kann so unter Verwendung kurzer synthetischer DNA-Oligonucleotide, die an den entstehenden Schnittstellen kurze einzelsträngige Übergänge aufweisen, oder durch chemische Synthese hergestellte Gene die gewünschte Nucleotidsequenz nach einem Fachmann geläufigen molekularbiologischen Methoden in ein geeignetes Plasmid cloniert werden (vgl. Maniatis et al., s.o.). Das DNA-Molekül wird dann aus dem Plasmid, in welchem es in einfacher oder mehrfacher Kopie vorliegen kann, durch Verdauung mit Restriktionsendonukleasen ausgeschnitten.

Die modifizierte mRNA, welche in der erfindungsgemäßen pharmazeutischen Zusammensetzung enthalten ist, kann darüber hinaus eine 5'-Cap-Struktur (ein modifiziertes Guanosin-Nucleotid) aufweisen. Als Beispiele von Cap-Strukturen können m7G(5')ppp (5'(A,G(5')ppp(5')A und G(5')ppp(5')G genannt werden.

Gemäß einer weiteren bevorzugten Ausführungsform der vorliegenden Erfindung enthält die modifizierte mRNA einen PolyA-Schwanz von mindestens 50 Nuclotiden, vorzugsweise mindestens 70 Nuclotiden, mehr bevorzugt mindestens 100 Nuclotiden, besonders bevorzugt mindestens 200 Nuclotiden.

Für eine effiziente Translation der mRNA ist weiterhin eine wirksame Bindung der Ribosomen an die Ribosomen-Bindungsstelle (Kozak-Sequenz: GCCGCCACCAUGG, das AUG bildet das Startcodon) erforderlich. Diesbezüglich ist festgestellt worden, daß ein erhöhter A/U-Gehalt um diese Stelle herum eine effizientere Ribosomen-Bindung an die mRNA ermöglicht.

Des weiteren ist es möglich, in die modifizierte mRNA eine oder mehrere sog. IRES (engl. "internal ribosomal entry side) einzufügen. Eine IRES kann so als alleinige Ribosomen-Bindungsstelle fungieren, sie kann jedoch auch zur Bereitstellung einer mRNA dienen, die mehrere Peptide bzw. Polypeptide codiert, die unabhängig voneinander durch die Ribosomen translatiert werden sollen ("multicistronische mRNA"). Beispiele erfindungsgemäß verwendbarer IRES-Sequenzen sind diejenigen aus Picornaviren (z.B. FMDV), Pestviren (CFFV), Polioviren (PV), Enzephalo-Myocarditis-Viren (ECMV), Maul-und-Klauenseuche-Viren (FMDV), Hepatitis-C-Viren (HCV), Klassisches-Schweinefieber-Viren (CSFV), Murines-Leukoma-Virus (MLV), Simean-Immundefizienz-Viren (SIV) oder Cricket-Paralysis-Viren (CrPV).

Gemäß einer weiteren bevorzugten Ausführungsform der vorligenden Erfindung weist die modifizierte mRNA in den 5'- und/oder 3'-nicht translatierten Bereichen Stabilisierungssequenzen auf, die befähigt sind, die Halbwertszeit der mRNA im Cytosol zu erhöhen.

Diese Stabilisierungssequenzen können eine 100%ige Sequenzhomologie zu natürlich vorkommenden Sequenzen, die in Viren, Bakterien und Eukaryoten auftreten, aufweisen, können aber auch teilweise oder vollständig synthetischer Natur sein. Als Beispiel für stabilisierende Sequenzen, die in der vorliegenden Erfindung verwendbar sind, können die nicht-translatierten Sequenzen (UTR) des β-Globingens, bspw. von *Homo sapiens* oder *Xenopus laevis,* genannt werden. Ein anderes Beispiel einer Stabilisierungssequenz weist die allgemeine Formel (C/U)CCANₓCCC(U/A)PyₓUC(C/U)CC auf, die im 3'UTR der sehr stabilen mRNA enthalten ist, die für α-Globin, α-(I)-Collagen, 15-Lipoxygenase oder für Tyrosin-Hydroxylase codiert (vgl. Holcik et al., Proc. Natl. Acad. Sci. USA 1997, 94: 2410 bis 2414). Selbstverständlich können derartige Stabilisierungssequenzen einzeln oder in Kombination miteinander als auch in Kombination mit anderen, einem Fachmann bekannten Stabilisierungssequenzen verwendet werden.

Zur weiteren Stabilisierung der modifizierten mRNA ist es außerdem bevorzugt, daß diese mindestens ein Analoges natürlich vorkommender Nucleotide aufweist. Dies beruht auf der Tatsache, daß die in den Zellen vorkommenden RNA-abbauenden Enzyme als Substrat vorzugsweise natürlich vorkommende Nucleotide erkennen. Durch Einfügen von Nucleotidanaloga kann daher der RNA-Abbau erschwert werden, wobei die Auswirkung auf die Translationseffizienz bei Einfügen von diesen Analoga, insbesondere in den codierenden Bereich der mRNA, einen positiven oder negativen Effekt auf die Translationseffizienz haben kann.

In einer keineswegs abschließenden Aufzählung können als Beispiele erfindungsgemäß verwendbarer Nucleotidanaloga Phosphoramidate, Phosphorthioate, Peptidnucleotide, Methylphosphonate, 7-Deazaguaonsin, 5-Methylcytosin und Inosin genannt werden. Die Herstellung derartiger Analoga sind einem Fachmann bspw. aus den US-Patenten 4,373,071, US 4,401,796, US 4,415,732, US 4,458,066, US 4,500,707, US 4,668,777, US 4,973,679, US 5,047,524, US 5,132,418, US 5,153,319, US 5,262,530 und 5,700,642 bekannt. Erfindungsgemäß können derartige Analoga in nicht-translatierten und translatierten Bereichen der modifizierten mRNA vorkommen.

Des weiteren kann der wirksame Transfer der modifizierten mRNA in die zu behandelnden Zellen bzw. den zu behandelnden Organismus dadurch verbessert werden, daß die modifizierte mRNA mit einem kationischen Peptid oder Protein assoziiert oder daran gebunden ist. Insbesondere ist dabei die Verwendung von Protamin als polykationischem, Nucleinsäure-bindenden Protein besonders wirksam. Des weiteren ist die Verwendung anderer kationischer Peptide oder Proteine, wie Poly-L-Lysin oder Histonen, ebenfalls möglich. Diese Vorgehensweise zur Stabilisierung der modifizierten mRNA ist in EP-A-1083232 beschrieben, deren diesbezüglicher Offenbarungsgehalt in die vorliegende Erfindung vollumfänglich eingeschlossen ist.

Bei der gentherapeutischen Verwendung der erfindungsgemäßen pharmazeutischen Zusammensetzung (bzw. bei der Verwendung der mRNA zur Gentherapie bzw. bei der Verwendung der mRNA zur Herstelllung einer pharmazeutischen Zusammensetzung zur Gentherapie) codiert die modifizierte mRNA für mindestens ein biologisch wirksames Peptid oder Polypeptid, welches in dem zu behandelnden Patienten bspw. nicht, nur unzureichend oder fehlerhaft gebildet wird. Daher sind Beispiele von der erfindungsgemäßen mRNA codierte Polypeptide Dystrophin, der Chloridkanal, der bei der cystischen Fibrose fehlerhaft verändert ist, Enzyme, die bei metabolischen Erkrankungen, wie Phenylketonurie, Galktosämie, Homocystinurie, Adenosindeaminasedefizienz usw., fehlen bzw. fehlerhaft sind, Enzyme, die an der Synthese von Neurotransmittern, wie Dopamin, Norepinephrin und GABA beteiligt sind, insbesondere Tyrosinhydroxylase und DOPA-Decarboxylase, α-1-Antitrypsin usw. Des weiteren kann die pharmazeutische Zusammensetzung zur Abgabe von Zelloberflächenrezeptoren und Molekülen, die an derartige Rezeptoren binden, verwendet werden, indem die in der pharmazeutischen Zusammensetzung enthaltene modifizierte mRNA für ein derartiges biologisch wirksames Protein bzw. Peptid codiert Beispiele derartiger Proteine, die extrazellulär wirken oder an Zelloberflächenrezeptoren binden, sind bspw. Gewebsplasminogenaktivator (TPA), Wachstumshormone, Insulin, Interferone, Granulocyten-Makrophagen-Koloniestimulisierungsfaktor (GM-CFS), Erythropoietin (EPO) usw. Durch Auswahl geeigneter Wachstumsfaktoren kann die pharmazeutische Zusammensetzung der vorliegenden Erfindung bspw. zur Geweberegeneration verwendet werden. Dadurch können Erkrankungen, die durch eine Gewebedegeneration gekennzeichnet sind, bspw. neurodegenerative Erkrankungen, wie Morbus Alzheimer, Morbus Parkinson usw., und andere degenerative Erkrankungen, bspw. Arthrose, behandelt werden. In diesen Fällen codiert die, insbesondere in der pharmazeutischen Zusammensetzung der vorliegenden Erfindung enthaltene, modifizierte mRNA vorzugsweise für Wachstumsfaktoren aus der TGF-β -Familie, wobei insbesondere EGF, FGF, PDGF, BMP, GDNF, BDNF, GDF und neurotrophe Faktoren, wie NGF, Neutrophine usw. zu nennen sind.

Ein weiterer Anwendungsbereich der vorliegenden Erfindung ist die Vakzinierung, d.h. die Verwendung der modifizierten mRNA zur Impfung bzw. die Verwendung der pharmazeutischen Zusamensetzung als Impfstoff bzw. die Verwendung der modifizierten mRNA zur Herstellung der pharmazeutischen Zusammesetzung zur Impfung. Die Vakzinierung beruht auf der Einbringung eines Antigens, im vorliegenden Fall der genetischen Information für das Antigen in Form der für das Antigen codierenden modifizierten mRNA, in den Organismus, insbesondere in die Zelle. Die in der pharmazeutischen Zusammensetzung enthaltene modifizierte mRNA wird in das Antigen translatiert, d.h. das von der modifizierten mRNA codierte Polypeptid bzw. antigene Peptid wird exprimiert, wodurch eine gegen dieses Polypeptid bzw. antigene Peptid gerichtete Immunantwort stimuliert wird. Bei der Vakzinierung gegen einen pathologischen Keim, d.h, ein Virus, ein Bakterium oder ein protozoologischer Keim, wird daher ein Oberflächenantigen eines derartigen Keims zur Vakzinierung mit Hilfe der erfindungsgemäßen pharmazeutischen Zusammensetzung, enthaltend die für das Oberflächenantigen codierende modifizierte mRNA, verwendet. Im Fall der Verwendung als genetische Vakzine zur Behandlung von Krebs wird die Immunantwort durch Einbringung der genetischen Information für Tumorantigene, insbesondere Proteine, die ausschließlich auf Krebszellen exprimiert werden, erreicht, in dem eine erfindungsgemäße pharmazeutische Zusammensetzung verabreicht wird, die eine für ein derartiges Krebsantigen codierende mRNA enthält. Dadurch wird das oder die Krebsantigen(e) im Organismus exprimiert, wodurch eine Immunantwort hervorgerufen wird, die wirksam gegen die Krebszellen gerichtet ist.

In ihrer Verwendung als Vakzine kommt die erfindungsgemäße pharmazeutische Zusammensetzung insbesondere zur Behandlung von Krebserkrankungen (wobei die modifizierte mRNA für ein tumorspezifisches Oberflächenantigen (TSSA) codiert), bspw. zur Behandlung von malignem Melanom, Kolon-Karzinom, Lymphomen, Sarkomen, kleinzelligem Lungenkarzinom, Blastomen usw., in Betracht. Spezifische Beispiele von Tumorantigenen sind u.a. 707-AP, AFP, ART-4, BAGE, β-Catenin/m, Bcr-abl, CAMEL, CAP-1, CASP-8, CDC27/m, CDK4/m, CEA, CT, Cyp-B, DAM, ELF2M, ETV6-AML1, G250, GAGE, GnT-V, Gp100, HAGE, HER-2/neu, HLA-A*0201-R170I, HPV-E7, HSP70-2M, HAST-2, hTERT (oder hTRT), iCE, KIAA0205, LAGE, LDLR/FUT, MAGE, MART-1/Melan-A, MC1R, Myosin/m, MUC1, MUM-1, -2, -3, NA88-A, NY-ESO-1, p190 minor bcr-abl, Pm1/RARα, PRAME, PSA, PSM, RAGE, RU1 oder RU2, SAGE, SART-1 oder SART-3, TEL/AML1, TPI/m, TRP-1, TRP-2, TRP-2/INT2 und WT1. Des weiteren wird die erfindungsgemäße pharmazeutische Zusammensetzung gegen Infektionserkrankungen (z.B. virale Infektionskrankheiten, wie AIDS (HIV), Hepatitis A, B oder C, Herpes, Herpes Zoster (Varizellen), Röteln (Rubeola-Virus), Gelbfieber, Dengue usw. (Flavi-Viren), Grippe (Influenza-Viren), hämorrhagische Infektionskrankheiten (Marburg- oder Ebola-Viren), bakterielle Infektionserkrankungen, wie Legionärskrankheit (Legionella), Magengeschwür (Helicobacter), Cholera (Vibrio), *E.coli*-Infektionen, Staphylokokken-Infektionen, Salmonellen-Infektionen oder Streptokokken-Infektionen (Tetanus), oder protozoologische Infektionserkrankungen (Maleria, Schlafkrankheit, Leishmaniose, Toxoplasmose, d.h. Infektionen durch Plasmodium, Trypanosomen, Leishmania und Toxoplasmen) eingesetzt. Vorzugsweise werden auch im Falle von Infektionserkrankungen die entsprechenden Oberflächenantigene mit dem stärksten antigenen Potenzial durch die modifizierte mRNA codiert. Bei den genannten Genen pathogener Keime bzw. Organismen, insbesondere bei viralen Genen, ist dies typischerweise eine sekretierte Form eines Oberflächenantigens. Des weiteren werden erfindungsgemäß bevorzugt für Polypeptide codierende mRNAs eingesetzt, wobei es sich bei den Polypeptiden um Polyepitope, bspw. der vorstehend genannten Antigene, insbesondere Oberflächenantigene von pathogenen Keimen bzw. Organismen oder Tumorzellen, vorzugsweise sekretierter Proteinformen, handelt.

Darüber hinaus kann die erfindungsgemäß modifizierte mRNA neben dem antigenen oder dem gentherapeutisch wirksamen Peptid bzw. Polypeptid auch mindestens einen weiteren funktionalen Abschnitt enthalten, der bspw. für ein die Immunantwort förderndes Cytokin (Monokin, Lymphokin, Interleukin oder Chemokin, wie IL-1, IL-2, IL-3, IL-4, IL-5, IL-6, IL-7, IL-8, IL-9, IL-10, IL-12, INF-α, INF-γ, GM-CFS, LT-α oder Wachstumsfaktoren, wie hGH, codiert.

Des weiteren kann zur Erhöhung der Immunogenizität die erfindungsgemäße pharmazeutische Zusammensetzung ein oder mehrere Adjuvanzien enthalten. Unter "Adjuvans" ist dabei jede chemische oder biologische Verbindung zu verstehen, die eine spezifische Immunantwort begünstigt. In Abhängigkeit der verschiedenen Arten von Adjuvanzien können diesbezüglich verschiedene Mechanismen in Betracht kommen. Bspw. bilden Verbindungen, die eine Endocytose der in der pharmazeutischen Zusammensetzung enthaltenen modifizierten mRNA durch dendritische Zellen (DC) fordern, eine erste Klasse von verwendbaren Adjuvanzien. Andere Verbindungen, welche die Reifung der DC erlauben, bspw. Lipopolysaccharide, TNF-α oder CD40-Ligand, sind eine weitere Klasse geeigneter Adjuvanzien. Allgemein kann jedes das Immunsystem beeinflussende Agens von der Art eines "Gefahrsignals" (LPS, GP96, Oligonucleotide mit dem CpG-Motiv) oder Cytokine, wie GM-CFS, als Adjuvans verwendet werden, welche es erlauben, eine Immunantwort gegen ein Antigen, das durch die modifizierte mRNA codiert wird, zu erhöhen und/oder gerichtet zu beeinflussen. Insbesondere sind dabei die vorstehend genannten Cytokine bevorzugt. Weitere bekannte Adjuvanzien sind Aluminiumhydroxid, das Freud'sche Adjuvans sowie die vorstehend genannten stabilisierenden kationischen Peptide bzw. Polypeptide, wie Protamin. Des weiteren sind Lipopeptide, wie Pam3Cys, ebenfalls besonders geeignet, um als Adjuvanzien in der pharmazeutischen Zusammmensetzung der vorliegenden Erfindung eingesetzt zu werden; vgl. Deres et al, Nature 1989, 342: 561-564.

Die erfindungsgemäße pharmazeutische Zusammensetzung enthält neben der modifizierten mRNA einen pharmazeutisch verträglichen Träger und/oder ein pharmazeutisch verträgliches Vehikel. Entsprechende Wege zur geeigneten Formulierung und Herstellung der erfindungsgemäßen pharmazeutischen Zusammensetzung sind bei "Remington's Pharmaceutical Sciences" (Mack Pub. Co., Easton, PA, 1980) offenbart, das vollinhaltlich Bestandteil der Offenbarung der vorliegenden Erfindung ist. Für die parenterale Verabreichung kommen als Trägerstoffe bspw. steriles Wasser, sterile Kochsalzlösungen, Polyalkylenglykole, hydrogenierte Naphthalen und insbesondere biokompatible Lactidpolymere, Lactid/Glycolidcopolymer oder Polyoxyethylen-/Polyoxypropylencopolymere in Betracht. Erfindungsgemäße Zusammensetzungen können Füllsubstanzen oder Substanzen, wie Lactose, Mannitol, Substanzen zur kovalenten Anknüpfung von Polymeren, wie z.B. Polyethylenglykol an erfindungsgemäße Inhibitoren, Komplexierung mit Metallionen oder Einschluß von Materialien in oder auf besondere Präparationen von Polymerverbindung, wie z.B. Polylactat, Polyglykolsäure, Hydrogel oder auf Liposomen, Mikroemulsion, Micellen, unilamellare oder multilamellare Vesikel, Erythrozyten-Fragmente oder Sphäroplasten, enthalten. Die jeweiligen Ausführungsformen der Zusammensetzungen werden abhängig vom physikalische Verhalten, beispielsweise in Hinblick auf die Löslichkeit, die Stabilität, Bioverfügbarkeit oder Abbaubarkeit gewählt. Kontrollierte oder konstante Freisetzung der erfindungsgemäßen Wirkstoffkomponente in der Zusammensetzung schließt Formulierungen auf der Basis lipophiler Depots ein (z.B. Fettsäuren, Wachse oder Öle). Im Rahmen der vorliegenden Erfindung werden auch Beschichtungen erfindungsgemäßer Substanzen oder Zusammensetzungen, enthaltend solche Substanzen, nämlich Beschichtungen mit Polymeren offenbart (z.B. Poloxamere oder Poloxaxnine). Weiterhin können erfindungsgemäßen Substanzen bzw. Zusammensetzungen protektive Beschichtungen, z.B. Proteaseinhibitoren oder Permeabilitätsverstärker, aufweisen. Bevorzugte Träger sind typischerweise wässrige Trägermaterialien, wobei Wasser zur Injektion (WFI) oder Wasser, gepuffert mit Phosphat, Citrat oder Acetat usw. verwendet wird, und der pH typischerweise auf 5,0 bis 8,0, vorzugsweise 6,0 bis 7,0, eingestellt wird. Der Träger bzw. das Vehikel wird zusätzlich vorzugsweise Salzbestandteile enthalten, z.B. Natriumchlorid, Kaliumchlorid oder andere Komponenten, welche die Lösung bspw. isotonisch machen. Weiterhin kann der Träger bzw. das Vehikel neben den vorstehend genannten Bestandteilen zusätzliche Komponenten, wie humanes Serumalbumin (HSA), Polysorbat 80, Zucker oder Aminosäuren, enthalten.

Die Art und Weise der Verabreichung und die Dosierung der erfindungsgemäßen pharmazeutischen Zusammensetzung hängen von der zu behandelnden Erkrankung und deren Fortschrittsstadium, wie auch dem Körpergewicht, dem Alter und dem Geschlecht des Patienten ab.

Die Konzentration der modifizierten mRNA in derartigen Formulierungen kann daher innerhalb eines weiten Bereichs von 1 µg bis 100 mg/ml variieren. Die erfindungsgemäße pharmazeutische Zusammensetzung wird vorzugsweise parenteral, bspw. intravenös, intraarteriell, subkutan, intramuskulär, dem Patienten verabreicht. Ebenso ist es möglich, die pharmazeutische Zusammensetzung topisch oder oral zu verabreichen.

Somit wird erfindungsgemäß auch ein Verfahren zur Behandlung der vorstehend genannten Krankheiten bzw. ein Impfverfahren zur Prävention der vorstehend genannten Erkrankungen bereitgestellt, welches das Verabreichen der erfindungsgemäßen pharmazeutischen Zusammensetzung an einen Patienten, insbesondere einen Menschen, umfasst.

Darüber hinaus wird ebenfalls ein Verfahren bereitgestellt, dass der Ermittlung der modifizierten Sequenz der in der erfindugsgemäßen pharmazeutischen Zusammensetzung enthaltenen mRNA dient. Dabei wird erfindugsgemäß die Adaptierung der RNA-Sequenzen mit zwei unterschiedlichen Optimierungszielen durchgeführt: Zum einen mit größtmöglichen G/C-Gehalt, zum anderen unter bestmöglicher Berücksichtigung der Häufigkeit der tRNAs gemäß Codon Usage. Im ersten Schritt des Verfahrens wird eine virtuelle Translation einer beliebigen RNA(oder DNA)-Sequenz durchgeführt, um die entsprechende Aminosäuresequenz zu generieren. Ausgehend von der Aminosäuresequenz wird eine virtuelle reverse Translation durchgeführt, die aufgrund des degenerierten genetischen Codes Wahlmöglichkeiten für die entsprechenden Codons liefert. Je nach geforderter Optimierung bzw. Modifizierung werden zur Auswahl der geeigneten Codons entsprechende Selektionslisten und Optimierungsalgorithmen verwendet. Die Ausführung der Algorithmen erfolgt typischerweise mit Hilfe einer geeigneten Software auf einem Computer. So wird die optimierte mRNA-Sequenz erstellt und kann bspw. mit Hilfe einer entsprechenden Anzeigevorrichtung ausgegeben und mit der usrpünglichen (Wildtyp-)Sequenz verglichen werden. Das gleiche gilt auch für die Häufigkeit der einzelnen Nucleotide. Die Änderungen gegenüber der ursprünglichen Nucleotidsequenz werden dabei vorzugsweise hervorgehoben. Weiter werden gemäß einer bevorzugten Ausführungsform in der Natur bekannte stabile Sequenzen eingelesen, welche die Grundlage für eine gemäß natürlichen Sequenzmotiven stabilisierte RNA bieten können. Es kann ebenfalls eine Sekundärstrukturanalyse vorgesehen werden, die anhand von Strukturberechnungen stabilisierende und instabilisierende Eigenschaften bzw. Bereiche der RNA analysieren kann.

### Die Figuren zeigen:

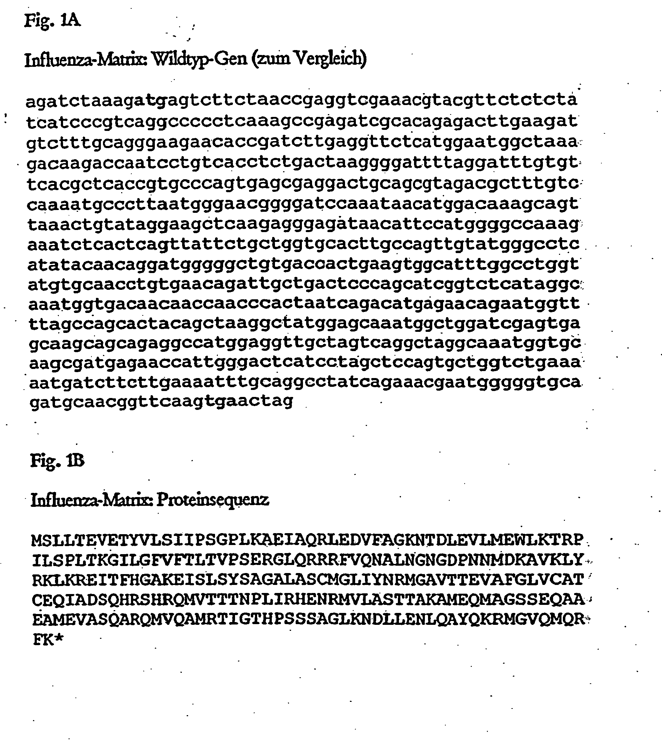

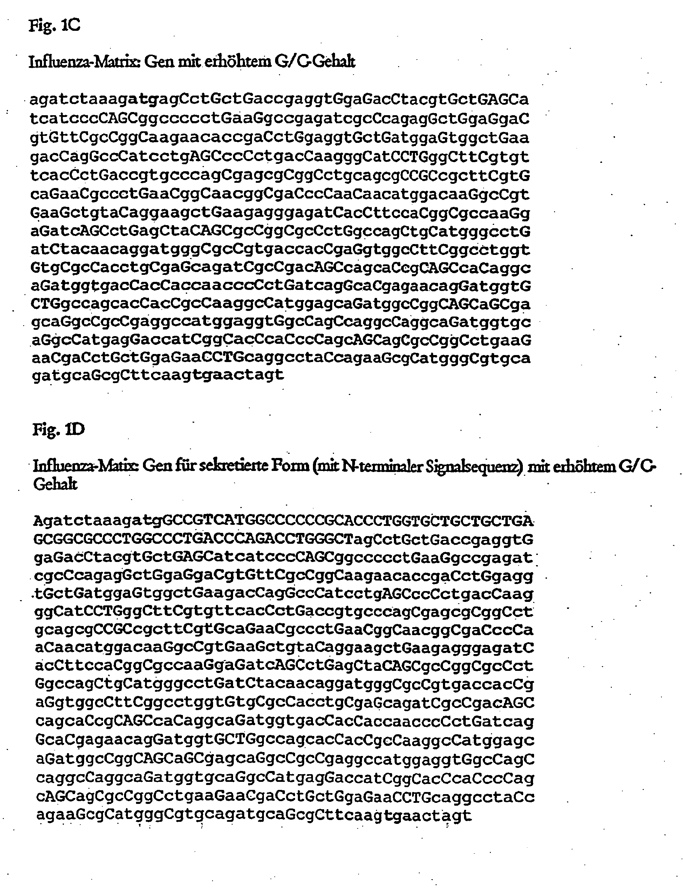

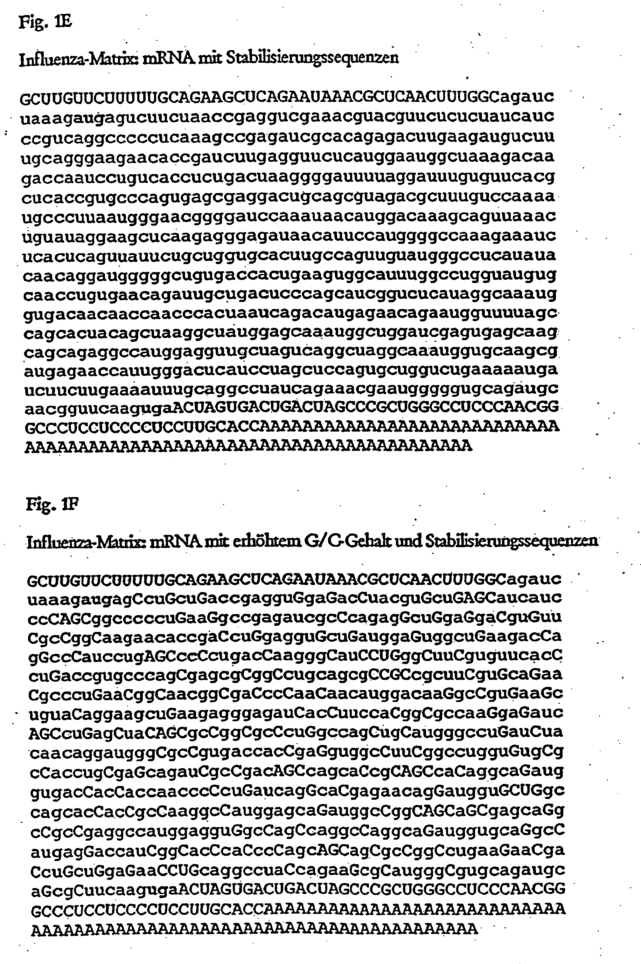

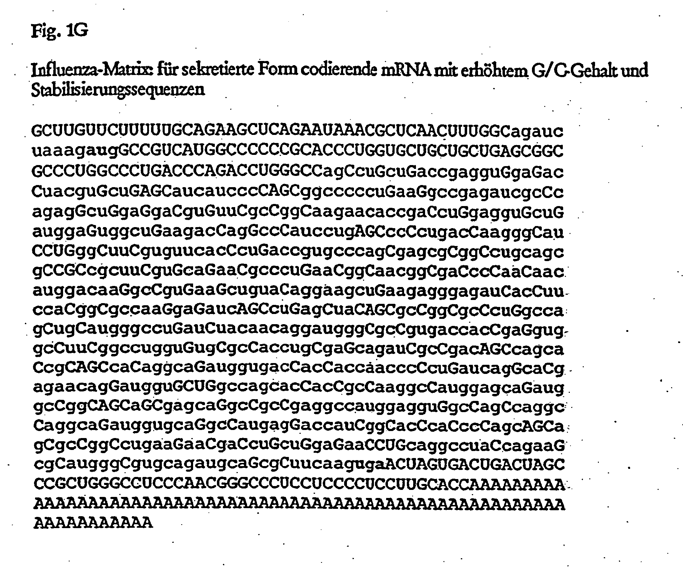

Fig. 1 zeigt Wildtyp- und modifizierte Sequenzen für das Influenza-Matrix-Protein.
Fig. 1A zeigt das Wildtyp-Gen und Fig. 1B zeigt die davon abgeleitete Aminosäuresequenz (Ein-Buchstaben-Code). Fig. 1C zeigt eine für das Influenza-Matrix-Protein codierende Gen-Sequenz, deren G/C-Gehalt gegenüber der Wildtyp-Sequenz erhöht ist. Fig. 1D zeigt die Sequenz eines Gens, das für eine sekretierte Form des Influenza-Matrix-Proteins codiert (einschließlich einer N-terminalen Signalsequenz), wobei der G/C-Gehalt der Sequenz gegenüber der Wildtyp-Sequenz erhöht ist. Fig. 1E zeigt eine für das Influenza-Matrix-Protein codierende mRNA, die im Vergleich zur Wildtyp-mRNA stabilisierende Sequenzen enthält. Fig. 1F zeigt eine für das Influenza-Matrix-Protein codierende mRNA, die neben stabilisierenden Sequenzen einen erhöhten G/C-Gehalt aufweist.
Fig. 1G zeigt ebenfalls eine modifizierte mRNA, die für die sekretierte Form des Influenza-Matrix-Proteins codiert und im Vergleich zum Wildtyp stabilisierende Sequenzen und einen erhöhten GC-Gehalt aufweist. In Fig. 1A und Fig. 1C bis 1G sind die Start- und Stop-Codons fett hervorgehoben. Gegenüber der Wildtyp-Sequenz von Fig. 1A veränderte Nucleotide sind in den Figuren 1C bis 1G in Großbuchstaben dargestellt

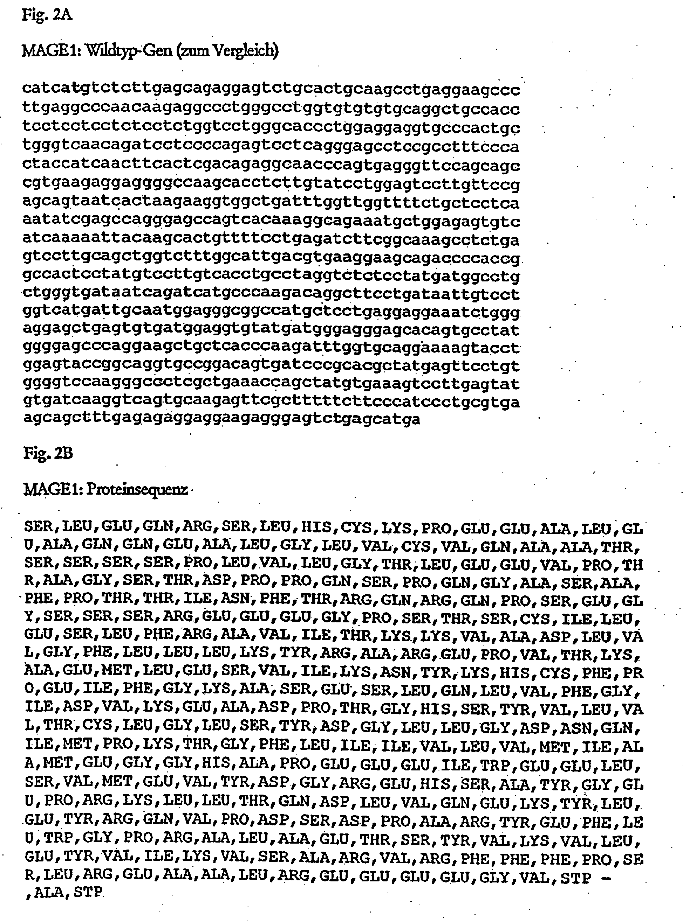

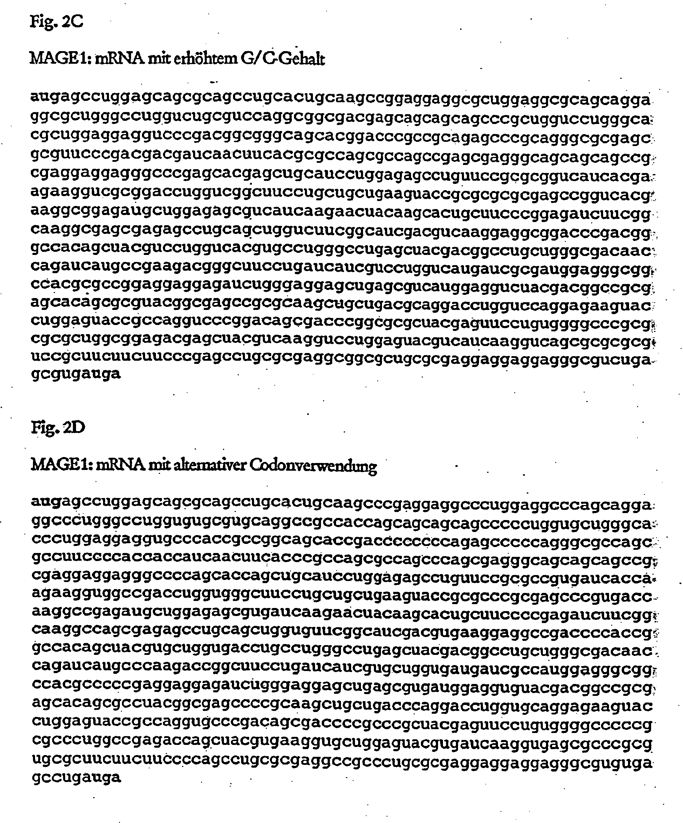

Fig. 2 zeigt Wildtyp- und erfindungsgemäß modifizierte Sequenzen, die für das Tumorantigen MAGE1 codieren.
Fig. 2A zeigt die Sequenz des Wildtyp-Gens und Fig. 2B die davon abgeleitete Aminosäuresequenz (Drei-Buchstaben-Code). Fig. 2C zeigt eine für MAGE1 codierende modifizierte mRNA, deren G/C-Gehalt gegenüber dem Wildtyp erhöht ist. Fig. 2D zeigt die Sequenz einer für MAGE1 codierenden modifizierten mRNA, bei der die Codon-Verwendung bezüglich der Codierung möglichst häufig in der Zelle vorkommender tRNA optimiert wurde. Start- und Stop-Codons sind jeweils fett gekennzeichnet.

Die folgenden Beispiele erläutern die vorliegende Erfindung näher, ohne sie einzuschränken.

### Beispiel 1

Als beispielhafte Ausführungsform des Verfahrens zu Ermittlung der Sequenz einer erfindungsgemäß modifizierten mRNA wurde ein Computerprogramm erstellt, das mit Hilfe des genetischen Codes bzw. dessen degenerativer Natur die Nucleotid-Sequenz einer beliebigen mRNA derart modifiziert, daß sich ein maximaler G/C-Gehalt in Verbindung mit der Verwendung von Codons, die für möglichst häufig in der Zelle vorkommende tRNAs codieren, ergibt, wobei die durch die modifizierte mRNA codierte Aminosäure-Sequenz gegenüber der nicht-modifizierten Sequenz identisch ist. Alternativ kann auch nur der G/C-Gehalt oder nur die Codonverwendung gegenüber der ursprünglichen Sequenz modifiziert werden.

Der Quellcode in Visual Basic 6.0 (eingesetzte Entwicklungsumgebung: Microsoft Visual Studio Enterprise 6.0 mit Servicepack 3) ist im Anhang angegeben.

### Beispiel 2

Mit Hilfe des Computerprogramms von Beispiel 1 wurde ein RNA-Konstrukt mit einer hinsichtlich Stabilisierung und Translationseffizienz optimierten Sequenz des lac-Z-Gens aus *E.coli* erstellt. Es konnte so ein G/C-Gehalt von 69% (im Vergleich zur Wildtyp-Sequenz von 51%; vgl. Kalnins et al., EMBO J. 1983, 2(4): 593-597) erzielt werden. Durch die Synthese überlappender Oligonucleotide, welche die modifizierte Sequenz aufweisen, wurde die optimierte Sequenz gemäß im Stand der Technik bekannter Verfahren hergestellt. Die endständigen Oligonucleotide wiesen die folgenden Restriktionsschnittstellen auf: Am 5'-Ende eine *Eco*RV*-* sowie am 3'-Ende eine *Bgl*II-Schnittstelle. Über Verdau mit *Eco*RV/*Bgl*II wurde die modifizierte lacZ-Sequenz in das Plasmid pT7Ts (GenBank-Zugriffsnummer U 26404; vgl. Lai et al., s.o.). pT7Ts enthält als nicht-translatierte Regionen Sequenzen aus dem β-Globingen von *Xenopus levis* jeweils an 5' und 3'. Das Plasmid wurde vor dem Einfügen der modifizierten lacZ-Sequenz mit den genannten Restriktionsenzymen geschnitten.

Das pT7Ts-lac-Z-Konstrukt wurde in Bakterien vermehrt und durch Phenol-Chloroform-Extraktion gereinigt. 2 µg des Konstrukts wurden *in vitro* mittels dem einem Fachmann bekannten Verfahren transkribiert, wodurch die modifizierte mRNA hergestellt wurde.

### Beispiel 3

Mit Hilfe des erfindungsgemäßen Computerprogramms von Beispiel 1 wurde das Gen für das Influenza-Matrix-Protein (Wildtyp-Sequenz vgl. Fig. 1A, abgeleitete Aminosäuresequenz Fig. 1 B) optimiert. Dadurch wurde die in Fig. 1C dargestellte G/C-reiche Sequenzvariante erstellt. Ebenfalls wurde eine für die sekretierte Form des Influenza-Matrix-Proteins codierende G/C-reiche Sequenz, die für eine N-terminale Signalsequenz codiert, ermittelt (vgl. Fig. 1D). Die sekretierte Form des Influenza-Matrix-Proteins hat den Vorteil, daß sie eine im Vergleich zur nicht-sekretierten Form erhöhte Immunogenizität aufweist.

Ausgehend von den optimierten Sequenzen wurden entsprechende mRNA-Moleküle entworfen. Die hinsichtlich G/C-Gehalt und Codonverwendung optimierte mRNA für das Influenza-Matrix-Protein wurde zusätzlich mit stabilisierenden Sequenzen im 5'- und 3'-Bereich (die Stabilisierungssequenzen stammen aus den 5'- bzw. 3'-UTRs des β-GlobinmRNA von *Xenupus laevis;* vgl. pT7Ts-Vektor in Lai et al., s.o.) ausgestattet (vgl. Fig. 1E und 1F). Desgleichen wurde auch die für die sekretierte Form des Influenza-Matrix-Proteins codierende mRNA im translatierten Bereich sequenzoptimiert und mit den genannten stabilisierenden Sequenzen versehen (vgl. Fig. 1G).

### Beispiel 4

Mit Hilfe des Computerprogramms von Beispiel 1 wurde die für das Tumorantigen MA-GE1 codierende mRNA modifiziert. Dadurch wurde die in Fig. 2C gezeigte Sequenz ermittelt, die im Vergleich zur Wildtyp-Sequenz (275 G, 244 G) einen um 24% höheren G/C-Gehalt aufweist (351 G, 291 C). Des weiteren wurde die Wildtyp-Sequenz durch alternative Codonverwendung hinsichtlich der Translationseffizienz aufgrund der Codierung von in der Zelle häufiger vorkommenden tRNAs verbessert (vgl. Fig. 2D). Durch die alternative Codonverwendung wurde der G/C-Gehalt ebenfalls um 24% erhöht.

### Anhang: Quelltext eines erfindungsgemäßen Computerprogramms

Curevac_Genetic_Controls mit folgenden Modulen
Name: Curevac_Genetic_Controls.vbp
Code:

Type=Control
Reference=*\G{00020430-0000-0000-C000-000000000046}#2.0#0#..\..\WINNT\System32\STDOLE2.TLB#OLE Automation
Object={0D452EE1-E08R101A-852E-02608C4DOBB4}# 2.0# 0; FM20.DLL
Object={F9043C88-F6F2-101A A3C9-08002B2F49FB}# 1.2# 0; COMDLG32.OCX
UserControl=Curevac_Amino.ctl
Startup="(None)"
HelpFile=""
Title ="Curevac Genetic Controls"
Command32=""
Name="Curevac_Genetic_Controls"
HelpContextID="0"
CompatibleMode ="1"
MajorVer=1
MinorVer=0
RevisionVer=0
AutoIncrementVer=0
ServerSupportFiles =0
VersionComments="the RNA people"
VersionCompanyName="CureVac GmbH"
VersionFileDescription="Controls for Handling of Nucleotids" VersionLegalCopyright="by Christian Klump"
VersionLegalTrademarks ="Curevac Genetic Controls(tm)" VersionProductName="Curevac Genetic Controls"
CompilationType =0
OptimizationType=0
FavorPentiumPro(tm) =0
CodeViewDebugInfo=0
NoAliasing=0
BoundsCheck=0
OverflowCheck=0
FlPointCheck=0
FDIVCheck=0
UnroundedFP=0
StartMode=1
Unattended=0
Retained=0
ThreadPerObject=0
MaxNumberOfThreads =1
ThreadingModel=1
DebugStartupOption=1
DebugStartupComponent=Curevac_Amino

Name: Curevac_Amino.ctl
Code:

VERSION 5.00
Object = "{0D452EE1-E08F-101A-852E-02608G4D0BB4}#2.0#0"; "FM20.DLL" Begin VB.UserControl Curevac_Amino
CanGetFocus = 0'False
   ClientHeight = 690
   ClientLeft = 0
   ClientTop = 0
   ClientWidth = 1200
   ScaleHeight = 690
   ScaleWidth = 1200
   Begin VB.Line linLower
      X1 = 0
      X2 = 1080
      Y1 = 600
      Y2 = 600
   End
   Begin VB.Line linUpper
      X1 = 120
      X2 = 1080
      Y1 = 0
      Y2 = 0
   End
   Begin MSForms.Label lblAminoAcid
      DragMode = 1 'Automatic
      Height = 255
      Left = 120
      TabIndex = 1
      Top = 360
      Width = 975
      Size = "1720;450"
      SpecialEffect = 1
      FontName = "Lucida Sans Unicode"
      FontEffects = 1073741826
      FontHeight = 165
      FontCharSet = 0
      FontPitchAndFamily= 2
      ParagraphAlign = 3
   End
   Begin MSForms.Label lblTriplett
      DragMode = 1 'Automatic
      Height = 255
      Left = 120
      TabIndex = 0
      Top = 120
      Width = 975
      Size = "1720;450"
      SpecialEffect = 1
      FontHeight = 165
      FontChatSet = 0
      FontPitchAndFamily= 2
      ParagraphAlign = 3
   End
End
Attribute VB_Name = "Curevac_Amino"
Attribute VB_GlobalNameSpace = False
Attribute VB_Creatable = True
Attribute VB_PredeclaredId = False
Attribute VB_Exposed = True
Option Explicit

Private msAAShortcut As String
Private msAAName As String
Private msBestTriplett As String
Private msSecondBest As String
Private msThirdBest As String
Private msTriplett As String
Private msBackColor As Long
Private mbShowOriginal As Boolean

Public Enum enuAminoAcid
   amaGlycin
   amaAlanin
   amaValin
   amaLeucin
   amaIsoLeucin
   amaPhenylalanin
   amaTyrosin
   amaTryptophan
   amaAsparaginAcid
   amaAsparagin
   amaGlutaminAcid
   amaGlutamin
   amaSerin
   amaThreonin
   amaCystein
   amaMethionin
   amaProlin
   amaHistidin
   amaLysin
   amaStop
   amaStart
End Enum

Private Sub Main()
   Call UserControl_Resize
End Sub

Public Sub Translation(ByVal sTriplett As String)
   msTriplett = sTriplett
   Select Case sTriplett
      Case "GGU", "GGC", "GGA", "GGG"
         msAAShortcut = "GLY"
      Case "GCU", "GCC", "GCA", "GCG"
         msAAShortcut = "ALA"
      Case "GUU", "GUC", "GUA", "GUG"
         rnsAAShortcut = "VAL"
      Case "UUA", "UUG", "CUU", "CUA", "CUG", "CUC"
         msAAShortcut = "LEU"
      Case "AUA", "AUU", "AUC"
         msAAShortcut = "ILE"
      Case "UUU", "UUC"
         msAAShortcut = "PHE"
      Case "UAU", "UAC"
         msAAShortcut = "TYR"
      Case "UGG"
         msAAShortcut = "TRP"
      Case "GAU", "GAC"
         msAAShortcut = "ASP"
      Case "AAU", "AAC"
         msAAShortcut = "ASN"
      Case "GAA", "GAG"
         msAAShortcut = "GLU"
      Case "CAA", "CAG"
         msAAShortcut = "GLN"
      Case "AGU", "AGC", "UCA", "UCU", "UCG", "UCC"
         msAAShortcut = "SER"
      Case "ACA", "ACU", "ACG", "ACC"
         rnsAAShortcut = "THR"
      Case "UGU", "UGC"
         msAAShortcut = "CYS"
      Case "AUG"
         msAAShortcut = "MET"
      Case "CCA", "CCU", "CCG", "CCC"
         msAAShortcut = "PRO"
      Case "CAU", "CAC"
         nisAAShortcut = "HIS"
      Case "AGA", "AGG", "CGA", "CGU", "CGG" , "CGC"
         msAAShortcut = "ARG"
      Case "AAA", "AAG"
         msAAShortcut = "LYS"
      Case "UAA", "UAG", "UGA"
         msAAShortcut = "STP"
   End Select
   Call Synthesis(msAAShortcut)
   Call Show
End Sub

Public Sub Synthesis(ByVal sShortCut As String)
   msAAShortcut = sShortCut 'nur falls die Sub extern angesprochen wird
   Select Case msAAShortcut
      Case "GLY"
      msAAName = "Glycin"
         msBestTriplett = "GGC"
         msSecondBest = "GGG"
         msBackColor = 8421631
      Case "ALA"
         msAAName = "Alanin"
         msBestTriplett = "GCG"
         msSecondBest = "GCC"
         msBackColor = 8454143
      Case "VAL"
         msAAName = "Valin"
         msBestTriplett = "GUC"
         msSecondBest = "GUG"
         msBackColor = 8454016
      Case "LEU"
         msAAName = "Leucin"
         msBestTriplett = "CUG"
         msSecondBest = "CUC"
         msBackColor = 16777088
      Case "ILE"
         msAAName = "Isoleucin"
         msBestTriplett = "AUC"
         msBackColor = 12615935
      Case "PHE"
         msAAName = "Phenylalanin"
         msBestTriplett = "UUC'
         msBackColor = 255
      Case "TYR"
         msAAName = "Tyrosin"
         msBestTriplett = "UAC"
         msBackColor = 4210816
      Case "TRP"
         msAAName = "Tryptophan"
         msBestTriplett = "UGG"
         msBackColor = 4227327
      Case "ASP"
         msAAName = "Asparaginsäure"
         msBestTriplett = "GAC"
         msBackColor = 8388863
      Case "ASN"
         msAAName = "Asparagin"
         msBestTriplett = "AAC"
         msBackColor = 4227200
      Case"GLU"
         msAAName = "Glutaminsäure"
         msBestTriplett = "GAG"
         msBackColor = 32768
      Case "GLN"
         msAAName = "Glutamin"
         msBestTriplett = "GGC"
         msBackColor = 8421440
      Case "SER"
         msAAName = "Serin"
         msBestTriplett = "AGC"
         msSecondBest = "UCG"
         msThirdBest = "UCC"
         msBackColor = 16512
      Case "THR"
         msAAName = "Threonin"
         msBestTriplett = "ACG"
         msBackColor = 16711680
      Case "CYS"
         msAAName = "Cystein"
         msBestTriplett = "UGC"
         msBackColor = 6932960
      Case "MET"
         msAAName = "Methionin"
         msBestTriplett = "AUG"
         msBackColor = 10417643
      Case "PRO"
         msAAName = "Prolin"
         msBestTriplett = "CCG"
         msSecondBest = "CCC"
         msBackColor = 12898746
      Case "HIS"
         msAAName = "Histidin"
         msBestTriplett = "CAC"
         msBackColor = 12898746
      Case "ARG"
         msAAName = "Arginin"
         msBestTriplett = "CGC"
         msSecondBest = "CGG"
         msBackColor = 6174925
      Case "LYS"
         msAAName = "Lysin"
         msBestTriplett = "AAG"
         msBackColor =14141641
      Case "STP"
         msAAName = "Stop"
         msBestTriplett = "UGA"
         msSecondBest = "UAG"
         msBackColor = 11332093
   End Select
End Sub

Public Sub Show()
   lblAminoAcid.Caption = msAAShortcut
   If mbShowOriginal = True Then
      lblTriplett.Caption = msTriplett
   Else
      lblTriplett.Caption = msBestTriplett
   End If
   lblAminoAcid.BackColor = msBackColor
   lblTriplett.BackColor = msBackColor
End Sub

Public Function ChooseBestGC(sShortCut As String)
   Select Case sShortCut
      Case "GLY"
         msAAName = "Glycin"
         msBestTriplett = "GGC"
         msSecondBest = "GGG"
         msBackColor = 8421631
      Case "ALA"
         msAAName = "Alanin"
         msBestTriplett = "GCG"
         msSecondBest = "GCC"
         nisBackGolor = 8454143
      Case "VAL"
         msAAName = "Valin"
         msBestTriplett = "GUC'
         msSecondBest = "GUG"
         msBackColor = 8454016
      Case "LEU"
         msAAName = "Leucin"
         msBestTriplett = "CUG"
         msSecondBest = "CUC"
         msBackColor = 16777088
      Case "ILE"
         msAAName = "Isoleucin"
         msBestTriplett = "AUC"
         msBackColor = 12615935
      Case "PHE"
         msAAName = "Phenylalanin"
         msBestTriplett = "UUC"
         msBackColor = 255
      Case "TYR"
         msAAName = "Tyrosin"
         msBestTriplett = "UAC"
         msBackColor = 4210816
      Case "TRP"
         msAAName = "Tryptophan"
         msBestTriplett = "UGG"
         msBackColor = 4227327
      Case "ASP"
         msAAName = "Asparaginsäure"
         msBestTriplett = "GAC"
         msBackColor = 8388863
      Case "ASN"
         msAAName = "Asparagin"
         msBestTriplett = "AAC"
         msBackColor = 4227200
      Case "GLU"
         msAAName = Glutaminsäure"
         msBestTriplett = "GAG"
         msBackColor = 16711808
      Case "GLN"
         msAAName = "Glutamin"
         msBestTriplett = "GGC"
         msBackColor = 12632256
      Case "SER"
         msAAName = "Serin"
         msBestTriplett = "AGC"
         msSecondBest = "UCG"
         msThirdBest = "UCC"
         msBackColor = 8421504
      Case "THR"
         msAAName = "Threonin"
         msBestTriplett = "ACG"
         msBackColor = &HD4C0FF
      Case "CYS"
         msAAName = "Cystein"
         msBestTriplett = "UGC"
         msBackColor = &HD5C0FF
      Case "MET"
         msAAName = "Methionin"
         msBestTriplett = "AUG"
         msBackColor = &HD6C0FF
      Case "PRO"
         msAAName = "Prolin"
         msBestTriplett = "CCG"
         msSecondBest = "CCC"
         msBackColor = &HD7C0FF
      Case "HIS"
         msAAName = "Histidin"
         msBestTriplett = "CAC"
         msBackColor = &HD8C0FF
      Case "ARG"
         msAAName = "Arginin"
         msBestTriplett = "CGC"
         msSecondBest = "CGG"
         msBackColor = &HD9C0FF
      Case "LYS"
         msAAName = "Lysin"
         msBestTriplett = "AAG"
         msBackColor = &HE0C0FF
      Case "STP"
         msAAName = "Stop"
         msBestTriplett = "UGA"
         msSecondBest = "UAG"
         msBackColor = &HE1C0FF
   End Select
End Function

Public Property Let ShowOriginal(ByVal bNewValue As Boolean)
   mbShowOriginal = bNewValue
End Property

Public Property Get ShowOriginal() As Boolean
   ShowOriginal = mbShowOriginal
End Property

Public Property Get AAShortcut() As String
   AAShortcut = msAAShortcut
End Property

Public Property Get AAName() As String
   AAName = msAAName
End Property

Public Property Get BestTriplett() As String
   BestTriplett = msBestTriplett
End Property

Public Property Get SecondBest() As String
   SecondBest = msSecondBest
End Property

Public Property Get ThirdBest() As String
   ThirdBest = ms ThirdBest
End Property

Public Property Get BackColor() As String
   BackColor = msBackColor
End Property

Private Property Get Triplett() As String
   Triplett = msTriplett
End Property

Private Sub UserControl_Resize()
Dim lOuterWidth As Long
Dim lDistance As Long
   lDistance = 30
   lOuterWidth = ScaleWidth + lDistance
   With lblTriplett
      - Top = 0
      - Left = lDistance / 2
      - Width = ScaleWidth - lDistance
      - Height = ScaleHeight / 2 + 30
   End With
   With lblAminoAcid
      .Top = ScaleHeight / 2 - 30
      .Left = lDistance / 2
      .Width = ScaleWidth - lDistance
      .Height = ScaleHeight / 2
   End With
   With linUpper
      .X1 = ScaleLeft
      .Y1 = 0
      .X2 = Scale Width
      .Y2 = 0
   End With
   With linLower
      .X1 = ScaleLeft
      .Y1 = ScaleHeight - 20
      .X2 = ScaleWidth
      .Y2 = ScaleHeight - 20
   End With
End Sub

Curevac_RNA_Optimizer mit folgenden Modulen
Name: Curevac_RNA_Optimizer.vbp
Code:

Type=Exe
Reference =*\G{00020430-0000-0000-C000-000000000046}# 2.0# 0# ..\..\WINNT\System32\STDOLE2.TLB# OLE Automation
Object={F9043C88-F6F2-101A-A3C9-08002B2F49FB}#1.2#0; COMDLG32.OCK
Module=basMain; basMain.bas
Form=mdiMain.frm
Object=*\ACurevac_Genetic_Controls.vbp
Object={38911DA0-E448-11D0-84A3-00DD01104159}# 1.1#0; COMCT332.OCX
Object={3B7C8863-D78F-101B-B9B5-04021O009402}# 1.2# 0; RICHTX32.OCX
Form=frmOutPut.frm
Form=frmStatistics.frm
Module=basPublics; basPublics.bas
Form=frmOptions.frm
Form=frmInput.frm
Form=frmDisplay.frm
Form=frmAbout.frm
Startup="mdiMain"
HelpFile=""
Title ="RNA-Optimizer"
Command32=""
Name="RNA_Optimizer"
HelpContextID="0"
CompatibleMode="0"
MajorVer=1
MinorVer=0
Revision Ver=0
AutoIncrement Ver=0
ServetSupportFiles =0
VersionComments ="the RNA people"
VersionCompanyName="CureVac GmbH"
VersionFileDescription="Application for Optimization of RNA"
VersionLegalCopyright="by Christian Klump"
VersionLegalTrademarks ="Curevac RNA-Optimizer(tm)"
VersionProductName="Curevac RNA-Optimizer"
Compilation Type =0
OptimizationType =0
FavorPentiumPro(tm) =0
CodeViewDebugInfo =0
NoAliasing=0
BoundsCheck=0
OverflowCheck=0
FlPointCheck=0
FDIVCheck=0
UnroundedFP=0
StartMode=0
Unattended=0
Retained=0
ThreadPerObject=0
MaxNumberOfThreads=1
DebugStartupOption=0

Name: basMain.bas
Code:
Attribute VB_Name = "basMain"
Option Explicit

Private Type udtAminoAcid
   sShortcut As String
   sName As String
   sBestTriplett As String
   sSecondBest As String
   sThirdBest As String
End Type
Private mastrAminoAcids() As udtAminoAcid
Public Sub RebuildChain(bShowOriginal As Boolean)
Dim iLoop As Integer
   For iLoop = 1 To glAminoCounter
      With frmDisplay.Cupevac_Amino1(iLoop)
         .ShowOriginal = bShowOriginal
         .Show
      End With
   Next iLoop
End Sub

Public Sub ResetPublics()
   gsOriginalCode = ""
   gsFormattedCode = ""
   glCodeLength = ""
End Sub

Public Sub BuildAminoChain(ByVal sTempCode As String, bGraphical As Boolean)
Dim lVerticalOffset As Long
Dim.lHorizontalOffset As Long
Dim lTop As Long
Dim asTripletts() As String
Dim sAminoChain As String
Dim lLoop As Long
   If bGraphical = True Then
      lTop = frmDisplay.optNucleotids(0).Height + 40
   End If
   ReDim mastrAminoAcids(glAminoCounter)
   asTripletts = Split(sTempCode)
   For lLoop = 0 To glAminoCounter - 1
      If bGraphical = True Then
      Load frmDisplay.Curevac_Amino1(lLoop + 1)
      With frmDisplay.Curevac_Amino1(lLoop + 1)
      Call.Translation(asTripletts(lLoop))
      .Left = lLoop *.Width - lHorizontalOffset
      .Top = lVerticalOffset + lTop
      If .Left + 2 * .Width > frmDisplay.Scale Width Then lVerticalOffset = lVerticalOffset +.Height + 150 lHorizontalOffset = lLoop *.Width +.Width
      End If

      sAminoChain = sAminoChain +.aaShortcut + "," .Visible = True
   End With
Else
   mastrAminoAcids(lLoop) = Translation(asTripletts(lLoop))
   sTest = sTest + mastrAminoAcids(iLoop).sName + ","
End If

Next
If bGraphical = True Then
   frmDisplay.Show
Else
   Call frmOutPut.Show
End If
   Debug.Print sAminoChain
End Sub

Private Function Translation(ByVal sTriplett As String) As udtAminoAcid
Dim stiTemp As udtAminoAcid
If mdiMain.optKindOfOptimize(0) = True Then
   'Optimization for Most GC
   Select Case sTriplett
      Case "GGU", "GGC", "GGA", "GGG"
         stiTemp.sShortcut = "GLY"
         stiTemp.sName = "Glycin"
         stiTemp.sBestTriplett = "GGC"
         stiTemp.sSecondBest = "GGG"
      Case "GCU", "GOC", "GCA", "GCG"
         stiTemp.sShortcut = "ALA"
         stiTemp.sName = "Alanin"
         stiTemp.sBestTriplett = "GCG"
         stiTemp.sSecondBest == "GCC"
      Case "GUU", "GUC", "GUA", "GUG"
         stiTemp.sShortcut = "VAL"
         stiTemp.sName = "Valin"
         stiTemp.sBestTriplett = "GUC"
         stiTemp.sSecondBest = "GUG"
      Case "UUA", "UUG", "CUU", "CUA", "CUG", "CUC"
         stiTemp.sShortcut = "LEU"
         stiTemp.sName = "Leucin"
         stiTemp.sBestTriplett = "CUG"
         stiTemp.sSecondBest = "CUC"
      Case "AUA", "AUU", "AUC"
         stiTemp.sShortcut = "ILE"
         stiTemp.sName = "Isoleucin"
         stiTemp.sBestTriplett = "AUC'
      Case "UUU", "UUC"
         stiTemp.sShortcut = "PHE"
         stiTemp.sName = "Phenylalanin"
         stiTemp.sBestTriplett = "UUC"
      Case "UAU", "UAC"
         stiTemp.sShortcut = "TYR"
         stiTemp.sName = "Tyrosin"
         stiTempsBestTriplett = "UAC"
      Case "UGG"
         stiTemp.sShortcut = "TRP"
         stiTemp.sName = "Tryptophan"
         stiTemp.sBestTriplett = "UGG"
      Case "GAU", "GAC"
         stiTemp.sShortcut = "ASP"
         stiTemp.sName = "Asparaginsäure"
         stiTemp.sBestTriplett = "GAC"
      Case "AAU", "AAC"
         stiTemp.sShortcut = "ASN"
         stiTemp.sName = "Asparagin"
         stiTemp.sBestTriplett = "AAC"
      Case "GAA", "GAG"
         stiTemp.sShortcut = "GLU"
         stiTemp.sName = "Glutaminsäure"
         stiTemp.sBestTriplett = "GAG"
      Case "CAA", "CAG"
         stiTemp.sShortcut = "GLN"
         stiTemp.sName = "Glutamin"
         stiTemp.sBestTriplett = "CAG"
      Case "AGU", "AGC", "UCA", "UCU", "UCG", "UCC"
         stiTemps.sShortcut = "SER"
         stiTemp.sName = "Serin"
         stiTemp.sBestTriplett = "AGC"
         stiTemp.sSecondBest = "UCG"
         stiTemp.sThirdBest = "UCC"
      Case "ACA", "ACU", "ACG", "ACC"
         stiTemp.sShortcut = "THR"
         stiTemp.sName = "Threonin"
         stiTemp.sBestTriplett = "ACG"
      Case "UGU", "UGC"
         stiTemp.sShortcut = "CYS"
         stiTemp.sName = "Cystein"
         stiTemp.sBestTriplett = "UGC"
      Case "AUG"
         stiTemp.sShortcut = "MET"
         stiTemp.sName = "Methionin"
         stiTemp.sBestTriplett = "AUG"
      Case "CCA", "CCU", "CCG", "CCC"
         stiTemp.sShortcut = "PRO"
         stiTemp.sName = "Prolin"
         stiTemp.sBestTriplett = "CCG"
         stiTemp.sSecondBest = "CCC"
      Case "CAU", "CAC"
         stiTemp.sShortcut = "HIS"
         stiTemp.sName = "Histidin"
         stiTemp.sBestTriplett = "CAC"
      Case "AGA", "AGG", "CGA", "CGU", "CGG", "CGC"
         stiTemp.sShortcut = "ARG"
         stiTemp.sName = "Arginin"
         stiTemp.sBestTriplett = "CGC"
         stiTemp.sSecondBest = "CGG"
      Case "AAA", "AAG"
         stiTemp.sShortcut = "LYS"
         stiTemp.sName = "Lysin"
         stiTemp.sBestTriplett = "AAG"
      Case "UAA", "UAG", "UGA"
         stiTemp.sShortcut = "STP"
         stiTemp.sName = "Stop"
         stiTempsBestTriplett = "UGA"
         stiTemp.sSecondBest = "UAG"
   End Select
   Translation = stiTemp
Else 'Optimization for best frequency
   Select Case sTriplett
      Case "GGU", "GGC", "GGA", "GGG"
         stiTemp.sShortcut = "GLY"
         stiTemp.sName = "Glycin"
         stiTemp.sBestTriplett = "GGC"
         stiTemp.sSecondBest = "GGU"
      Case "GCU", "GCC", "GCA", "GCG"
         stiTemp.sShortcut = "ALA"
         stiTemp.sName = "Alanin"
         stiTemp.sBestTriplett = "GCC"
         stiTemp.sSecondBest = "GCU"
      Case "GUU", "GUC", "GUA", "GUG"
         stiTemp.sShortcut = "VAL"
         stiTemp.sName = "Valin"
         stiTemp.sBestTriplett = "GUG"
         stiTemp.sSecondBest = "GUC"
      Case "UUA", "UUG", "CUU", "CUA", "CUG", "CUC"
         stiTempsShortcut = "LEU"
         stiTemp.sName = "Leucin"
         stiTemp.sBestTriplett = "CUG"
         stiTemp.sSecondBest = "CUC"
      Case "AUA", "AUU", "AUC'
         stiTemp.sShortcut = "ILE"
         stiTemp.sName = "Isoleucin"
         stiTemp.sBestTriplett = "AUC"
      Case "UUU", "UUC"
         stiTemp.sShortcut = "PHE"
         stiTemp.sName = "Phenylalanin"
         stiTempsBestTriplett = "UUC"
      Case "UAU", "UAC"
         stiTemp.sShortcut = "TYR"
         stiTemp.sName = "Tyrosin"
         stiTemp.sBestTriplett = "UAC'
      Case "UGG"
         stiTemp.sShortcut = "TRP"
         stiTemp.sName = "Tryptophan"
         stiTemp.sBestTriplett = "UGG"
      Case "GAU", "GAC"
         stiTemp.sShortcut = "ASP"
         stiTemp.sName = "Asparaginsäure"
         stiTemp.sBestTriplett = "GAC"
      Case "AAU", "AAC"
         stiTemp.sShortcut = "ASN"
         stiTemp.sName = "Asparagin"
         stiTemp.sBestTriplett = "AAC"
      Case "GAA", "GAG"
         stiTemp.sShortcut = "GLU"
         stiTemp.sName = "Glutaminsäure"
         stiTemp.sBestTriplett = "GAG"
      Case "CAA", "CAG"
         stiTemp.sShortcut = "GLN"
         stiTemp.sName = "Glutamin"
         stiTemp.sBestTriplett = "CAG"
      Case "AGU", "AGC", "UCA", "UCU", "UCG","UCC"
         stiTemp.sShortcut = "SER"
         stiTemp.sName = "Serin"
         stiTemp.sBestTriplett = "AGC"
         stiTemp.sSecondBest = "UCC"
         stiTemp.sThirdBest = "UCU"
      Case "ACA", "ACU", "ACG", "ACC"
         stiTemp.sShortcut = "THR"
         stiTemp.sName = "Threonin"
         stiTemp.sBestTriplett = "ACC"
      Case "UGU", "UGC"
         stiTemp.sShortcut = "CYS"
         stiTemp.sName = "Cystein"
         stiTemp.sBestTriplett = "UGC"
      Case "AUG"
         stiTemp.sShortcut = "MET"
         stiTemp.sName = "Methionin"
         stiTemp.sBestTriplett = "AUG"
      Case "CCA", "CCU", "CCG", "CCC"
         stiTemp.sShortcut = "PRO"
         stiTemp.sName = "Prolin"
         stiTemp.sBestTriplett = "CCC"
         stiTemp.sSecondBest ="CCU"
      Case "CAU', "CAC"
         stiTemp.sShortcut = "HIS"
         stiTemp.sName = "Histidin"
         stiTemp.sBestTriplett = "CAC"
      Case "AGA", "AGG", "CGA", "CGU", "CGG", "CGC'
         stiTemp.sShortcut = "ARG"
         stiTemp.sName = "Arginin"
         stiTemp.sBestTriplett = "CGC"
         stiTemp.sSecondBest = "AGG"
      Case "AAA", "AAG"
         stiTemp.sShortcut = "LYS"
         stiTemp.sName = "Lysin"
         stiTemp.sBestTriplett = "AAG"
      Case "UAA", "UAG", "UGA"
         stiTemp.sShortcut = "STP"
         stiTemp.sName = "Stop"
         stiTemp.sBestTriplett = "UGA"
         stiTempsSecondBest = "UAG"
   End Select
   Translation = stiTemp
End If

End Function
Public Function ReverseTranscription() As String
   Dim sTempCode As String
   Dim iLoop As Integer
   For iLoop = 0 To glAminoCounter - 1
      sTempCode = sTempCode + mastrAminoAcids(iLoop).sBestTriplett
   Next
   Reverse Transcription = sTempCode
End Function

Public Sub CountBases(ByVal sTempCode As String)
Dim lLoop As Long
Dim sFormattedCode As String
Dim sActualBase As String
Dim lCodeLength As Long
   lCodeLength = Len(sTempCode)
   For lLoop = 1 To CLng(lCodeLength)
      sActualBase = Mid(sTempCode, lLoop,1)
      Select Case sActualBase
      Case "A", "a"
         glOptAdenin = glOptAdenin + 1
      Case "U", "u", "T", "t"
         glOptThymin = glOptThymin + 1
      Case "G" "g"
         glOptGuanin = glOptGuanin + 1
      Case "C", "c"
         glOptCytosin = glOptCytosin + 1
   End Select
   Next lLoop
End Sub

Name: basPublics.bas
Code:
Attribute VB_Name = "basPublics"
Option Explicit

Public gsOriginalCode As String
Public gsFormattedCode As String
Public glCodeLength As Long

Public glAminoCounter As Long
Public glAdenin As Long
Public glThymin As Long
Public glGuanin As Long
Public glCytosin As Long
Public glOptAdenin As Long
Public glOptThymin As Long
Public glOptGuanin As Long
Public glOptCytosin As Long

Public gbSequenceChanged As Boolean

Name: mdiMain.frm
Code:

VERSION 5.00
Object = "{38911DA0-E448-11D0-84A3-00DD01104159}# 1.1#0"; "COMCT32.OCX"
Begin VB.MDIForm mdiMain
   BackColor = &H00FFFFFF&
   Caption = "Curevac_RNA_Analyzer"
   ClientHeight = 8745
   ClientLeft = 165
   ClientTop = 735
   ClientWidth = 12255
   Icon = "mdiMain.frx":0000
   LinkTopic = "MDIForm1"
   Picture = "mdiMain.frx ":058A
   StartUpPosition = 3 'Windows Default
   WindowState = 2 'Maximized
   Begin ComCtl3.CoolBar cbarMain
      Align = 1 'Align Top
      Height = 885
      Left = 0
      TabIndex = 0
      Top = 0
      Width = 12255
      _ExtentX = 21616
      _ExtentY = 1561
      BandCount = 1
      BandBorders = 0 'False
      _CBWidth = 12255
      _CBHeight = 885
      _Version = "6.7.8988"
      MinHeight1 = 825
      Width1 = 4995
      FixedBackground1 = 0 'False
      NewRow1 = 0 'False
      Begin VB.OptionButton optKindOfOptimize
         Caption = "Best Frequency"
         Height = 255
         Index = 1
         Left = 4920
         TabIndex = 7
         Top = 480
         Value = -1 True
         Width = 1575
      End
      Begin VB.OptionButton optKindOfOptimize
         Caption = "Best GC"
         Height = 255
         Index = 0
         Left = 4920
         TabIndex = 6
         Top = 120
         Width = 1575
      End
      Begin VB.CommandButton cmdShowInput
         Caption = "Input"
         Height = 765
         Left = 0
         Picture = "mdiMain.frx'';0C2A
         Style = 1 'Graphical
         TabIndex = 5
         Top = 0
         Width = 840
      End
      Begin VB.CommandButton cmdShowOptions
         Caption = "Options"
         Enabled = 0 'False
         Height = 760
         Left = 3480
         Picture = "mdiMain.frx":0F34
         Style = 1 'Graphical
         TabIndex = 4
         Top = 0
         Width = 735
      End
      Begin VB.CommandButton cmdShowStatistics
         Caption = "Statistics"
         Enabled = 0 'False
         Height = 760
         Left = 2640
         Picture = "mdiMain.frx":131E
         Style = 1 'Graphical
         TabIndex = 3
         Top = 0
         Width = 735
      End
      Begin VB.CommandButton cmdShowDisplay
         Caption = "Display"
         Enabled = 0 'False
         Height = 760
         Left = 1800
         Picture = "mdiMain.frx":1628
         Style = 1 'Graphical
         TabIndex = 2
         Top = 0
         Width = 735
      End
      Begin VB.CommandButton cmdShowOutput
         Caption = "Output"
         Enabled = 0 'False
         Height = 760
         Left = 960
         Picture = "mdiMain.frx":20E2
         Style = 1 'Graphical
         TabIndex = 1
         Top = 0
         Width = 735
      End
   End
   Begin VB.Menu mnuMain
      Caption = "&Input..."
      Index = 0
   End
   Begin VB.Menu mnuMain
      Caption = "&Results"
      Index = 1
      Begin VB.Menu mnuResults
         Caption = "&Output..."
         Enabled = 0 'False
         Index = 0
      End
      Begin VB.Menu mnuResults
         Caption = "&Display..."
         Enabled = 0 'False
         Index = 1
      End
      Begin VB.Menu mnuResults
         Caption = "&Statistics..."
         Enabled = 0 'False
         Index = 2
      End
   End
   Begin VB.Menu mnuMain
      Caption = "E&xtras"
      Index = 4
      Begin VB.Menu mnuExtras
         Caption = "&Language"
         Index = 0
         Begin VB.Menu mnuLanguage
            Caption = "English"
            Checked = -1 'True
            Index = 0
      End
      Begin VB.Menu mnuLanguage
         Caption = "&German"
         Enabled = 0 'False
         Index = 1
      End
      Begin VB.Menu mnuLanguage
         Caption = "&French"
         Enabled = 0 'False
         Index = 2
      End
   End
   Begin VB.Menu mnuExtras
      Caption = "&Options..."
      Enabled = 0 'False
      Index = 1
   End
   Begin VB.Menu mnuExtras
      Caption = "-"
      Index = 2
   End
   Begin VB.Menu mnuExtras
      Caption = "&About"
      Index = 3
   End
End
Begin VB.Menu mnuMain
   Caption = "&Windows"
   Index = 5
   WindowList = -1 'True
   Begin VB.Menu mnuWindows
      Caption = "Tile &Horizontally"
      Index = 0
   End
   Begin VB.Menu mnuWindows
      Caption = "Tile &Vertically"
      Index = 1
   End
      Begin VB.Menu mnuWindows
      Caption = "&Cascade"
      Index = 2
   End
End
Begin VB.Menu mnuMain
   Caption = "&Exit"
   Index = 6
End

End
Attribute VB_Name = "mdiMain"
Attribute VB_GlobalNameSpace = False
Attribute VB_Creatable = False
Attribute VB_PredeclaredId = True
Attribute VB_Exposed = False
Option Explicit

Private Sub cmdShowDisplay_Click()
   Call frmDisplay.Show
End Sub

Private Sub cmdShowInput_Click()
   Call frmInput.Show
End Sub

Private Sub cmdShowOptions_Click()
   Call frmOptions.Show
End Sub

Private Sub cmdShowOutput_Click()
   Call frmOutPut.Show
End Sub

Private Sub cmdShowStatistics_Click()
   Call frmStatistics.Show
End Sub

Private Sub MDIForm_Load()
   Call InitControls
   Call frmInput.Show
End Sub

Private Sub InitControls()

Dim lNextLeft As Long
Const lSpace As Long = 60
Const lButtonWidth As Long = 850
Const lButtonHeight As Long = 770
   lNextLeft = lSpace + lButton Width
   With cmdShowInput
      - Top = lSpace
      - Left = lSpace
      - Width - lButtonWidth
      - Height = lButtonHeight
   End With
   With cmdShowOutput
      .Top = lSpace
      .Left = lNextLeft + lSpace
      .Width = lButton Width
      .Height = lButtonHeight
   End With
   With cmdShowDisplay
      .Top = lSpace
      .Left = lNextLeft * 2 + lSpace
      .Width = lButton Width
      .Height = lButtonHeight
   End With
   With cmdShowStatistics
      .Top = lSpace
      .Left = lNextLeft * 3 + lSpace
      .Width = lButtonWidth
      .Height = lButtonHeight
   End With
   With cmdShowOptions
      .Top = lSpace
      .Left = lNextLeft* 4 + lSpace
      .Width = lButtonWidth
      .Height = lButonHeight
   End With
   cbarMain.Height = lButtonHeight + 2 * lSpace
End Sub

Private Sub nmuMain_Click(Index As Integer)
   Select Case Index
      Case 0
         Call frmInput.Show
      Case 6
         Unload Me
   End Select
End Sub

Private Sub nmuResults_Click(Index As Integer)
   Select Case Index
      Case 0 'Output
         Call frmOutPut.Show
      Case 1
         Call frmDisplay.Show
      Case 2 'Statistische Auswertung
         ' Call frmStatistics.Show
   End Select
End Sub

Private Sub mnuExtras_Click(Index As Integer)
   Select Case Index
      Case 0 'Output
      Case 1
         Call frmDisplay.Show
      Case 3
         Call frmAbout.Show(vbModal)
   End Select
End Sub

Private Sub mnuWindows_Click(Index As Integer)
   Select Case Index
      Case 0 'Horizontal
         Me.Arrange (vbTileHorizontal)
      Case 1 'Vertikal
         Me.Arrange (vbTile Vertical)
      Case 2 'Kaskadieren
         Me.Arrange (vbCascade)
   End Select
End Sub

Name: frmInput.frm
Code:

VERSION 5.00
Object = "{F9043C88-F6F2-101A-A3C9-08002B2F49FB}# 1.2#0"; "COMDLG32.OCX"
Object = "{3B7C8863-D78F-101B-B9B5-040210O09402}# 1.2#0"; "RICHTX32.OCX"
Begin VB.Form frmInput
   Caption = "Input"
   ClientHeight = 5730
   ClientLeft = 60
   ClientTop = 345
   ClientWidth = 13200
   Icon = "frmInput.frx":0000
   LinkTopic = "Form1''
   MDIChild = -1 True
   ScaleHeight = 5730
   ScaleWidth = 13200
   WindowState = 2 'Maximized
   Begin RichTextLib.RichTextBox txtFormatted
      Height = 1815
      Left = 120
      TabIndex = 3
      Top = 360
      Width = 12735
      _ExtentX = 22463
      _ExtentY = 3201
      _Version = 393217
      BorderStyle = 0
      Enabled = -1 'True
      ScrollBars = 2
      DisableNoScroll = -1 'True
      TextRTF = $"frmInput.frx":030A
      BeginPropertyFont {0BE35203-8F91-11CE-9DE3-00AA004BB851}
         Name = "Fixedsys"
         Size = 9
         Charset = 0
         Weight = 400
         Underline = 0 'False
         Italic = 0 'False
         Strikethrough = 0 'False
      EndProperty
   End
   Begin VB.OptionButton optSequence
      Caption = "Formatted"
      Height = 255
      Index = 1
      Left = 2760
      Style = 1 'Graphical
      TabIndex = 2
      Top = 0
      Value = -1 True
      Width = 855
   End
   Begin VB.OptionButton optSequence
      Caption = "Original"
      Height = 255
      Index = 0
      Left = 1920
      Style = 1 'Graphical
      TabIndex = 1
      Top = 0
      Width = 855
   End
   Begin VB.CommandButton cmdLoad
      Caption = "Load Sequence"
      Height = 285
      Left = 0
      TabIndex = 0
      Top = 0
      Width = 1695
   End
   Begin MSComDlg.CommonDialog CommonDialog1
      Left = 3840
      Top = -120
      _ExtentX = 847
      _ExtentY = 847
      _Version = 393216
   End
End
Attribute VB_Name = "frmInput"
Attribute VB_GlobalNameSpace = False
Attribute VB_Creatable = False
Attribute VB_PredeclaredId = True
Attribute VB_Exposed = False
Option Explicit

Private Sub Form_Activate()
   Me.ZOrder
   Call InitControls
End Sub

Public Sub LoadSequence()

Dim sFile As String
Dim sTempCode As String
   On Error GoTo Errorhandler
   With CommonDialog1
      .FileName = App.Path & "\SampleRNA.txt"
      .CancelError = True
      Call .ShowOpen
   End Wth
   If Len(CommonDialog1.FileName) > 0 Then
      gbSequenceChanged = True
      gsFormattedCode =""
      gsOriginalCode = ""
      sFile = CommonDialog1.FileName
      Open sFile For Input As # 1
      Input # 1, gsOriginalCode
      Close # 1
      Call FormatText
      Call FillSequence

      mdiMain.cmdShowDisplay.Enabled = True
      mdiMain.cmdShowOutput.Enabled = True
      mdiMain.cmdShowStatistics.Enabled = True
   End If
Exit Sub
Errorhandler:
Dim lAnswer As Long
   If Err.Number <> cdlCancel Then
      lAnswer = MsgBox("Datei konnte nicht geladen werden", vbRetryCancel, "Dateiproblem")
      If lAnswer = vbRetry Then
         Resume
      End If
   End If
End Sub

Private Sub FormatText()
Dim iLoop As Integer
Dim sActualBase As String
   glCodeLength = Len(gsOriginalCode)
   For iLoop = 0 To glCodeLength - 1
      sActualBase = Mid(gsOriginalCode, iLoop + 1,1)
      If iLoop Mod 3 = 0 Then
         gsFormattedCode = gsFormattedCode + ""
         glAminoCounter = glAminoCounter + 1
      End If
      Select Case sActualBase
         Case "A", "a"
            gsFormattedCode = gsFormattedCode + "A"
            glAdenin = glAdenin + 1
         Case "U', "u", "T", "t"
            gsFormattedCode = gsFormattedCode + "U"
            glThymin = glThymin + 1
         Case "G" "g"
            gsFormattedCode = gsFormattedCode + "G"
            glGuanin = glGuanin + 1
         Case "C", "c"
            gsFormattedCode = gsFormattedCode + "C"
            glCytosin = glCytosin + 1
      End Select
   Next iLoop
   gsFormattedCode = Trim(gsFormattedCode)
End Sub

Private Sub FillSequence()
   If optSequence(0).Value = True Then
      txtFormatted.Text = gsOriginalCode
   Else
      txtFormatted.Text = gsFormattedCode
   End If
End Sub

Private Sub cmdShowOutput_Click()
   Call frmOutPut.Show
End Sub

Private Sub cmdLoad_Click()
   Call LoadSequence
End Sub

Private Sub InitControls()

Const lSpace As Long = 40
Const lButtonHeight As Long = 285
   With cmdLoad
      .Top = lSpace
      .Height = lButtonHeight
      .Left = lSpace
   End With
   With optSequence(0)
      .Top = lSpace
      .Height = lButtonHeight
      .Left = cmdLoad.Left + cmdLoad.Width + lSpace + 200
   End With
   With optSequence(1)
      .Top = lSpace
      .Height = lButtonHeight
      .Left = optSequence(0).Left + optSequence(0).Width
   End With
   With txtFormatted
      .Top = cmdLoad.Height + 2* lSpace
      .Left = lSpace
      .Width = Me.ScaleWidth
      .Height = MeScaleHeight - txtFormatted.Top
   End With
End Sub

Private Sub optSequence_Click(Index As Integer)
   Call FillSequence
End Sub

Name: frmOutPut.frm
Code:

VERSION 5.00
Object = "{3B7C8863-D78F-101B-B9B5-04021CD09402}1.2#0"; "RICHTX32.OCX"
Begin VB.Form frmOutPut
   Caption = "OutPut"
   ClientHeight = 9120
   ClientLeft = 60
   ClientTop = 345
   ClientWidth = 10215
   Icon = "frmOutPut.frx":0000
   LinkTopic = "Form1"
   MDIChild = -1 'True
   ScaleHeight = 9120
   ScaleWidth = 10215
   Begin RichTextLib.RichTextBox txtOptimized
      Height = 2655
      Left = 0
      TabIndex = 0
      Top = 480
      Width = 9855
      _ExtentX = 17383
      _ExtentY = 4683
      _Version = 393217
      Enabled = -1 'True
      ScrollBars = 2
      DisableNoScroll = -1 'True
      TextRTF = $"frmOutPut.frx":0442
      BeginPropertyFont {0BE35203-8F91-11CE-9DE3-00AA004BB851}
         Name = "Fixedsys"
         Size = 9
         Charset = 0
         Weight = 400
         Underline = 0 'False
         Italic = 0 'False
         Strikethrough = 0 'False
      EndProperty
   End
End
Attribute VB_Name = "frmOutPut"
Attribute VB_GlobalNameSpace = False
Attribute VB_Creatable = False
Attribute VB_PredeclaredId = True
Attribute VB_Exposed = False
Option Explicit

Private Sub Form_Activate()
   Me.ZOrder
   Call InitControls
   If gbSequenceChanged = True Then
      Call BuildAminoChain(gsFormattedCode, False) End If
   gbSequenceChanged = False
   txtOptimized.Text = LCase(ReverseTranscription)
End Sub

Private Sub InitControls()

Const lSpace As Long = 40
   With txtOptimized
      .Top = lSpace
      .Left = lSpace
      Width = Me.ScaleWidth + lSpace
      Height = Me.ScaleHeight + lSpace
   End With
End Sub

Name: frmDisplay.frm
Code:

VERSION 5.00
Object = "*\ACurevac_Genetic_Controls.vbp"
Begin VB.Form frmDisplay
   Caption = "Display"
   ClientHeight = 8205
   ClientLeft = 60
   ClientTop = 345
   CiientWidth = 8745
   Icon = "frmDisplay.frx":0000
   LinkTopic = "Form1"
   MDIChild = -1 True
   ScaleHeight = 8205
   ScaleWidth = 8745
   ShowInTaskbar = 0 'False
   Begin VB.OptionButton optNucleotids
      Caption = "Original"
      Height = 255
      Index = 0
      Left = 0
      Style = 1 'Graphical
      TabIndex = 1
      Top = 0
      Width = 855
   End
   Begin VB.OptionButton optNucleotids
      Caption = "Optimized"
      Height = 255
      Index = 1
      Left = 840
      Style = 1 'Graphical
      TabIndex = 0
      Top = 0
      Value = -1 'True
      Width = 855
   End
   Begin Curevac_Genetic_Controls.Curevac_Amino Curevac_Amino1
      Height = 495
      Index = 0
      Left = 240
      Top = 360
      Visible = 0 'False
      Width = 735
      _ExtentX = 1296
      _ExtentY = 873
   End
End
Attribute VB_Name = "frmDisplay"
Attribute VB_GlobalNameSpace = False
Attribute VB_Creatable = False
Attribute VB_PredeclaredId = True
Attribute VB_Exposed = False
Option Explicit

Private Sub Form_Activate()
   Me.ZOrder
   If gbSequenceChanged = True Then
      Call BuildAminoChain(gsFormattedCode, True)
   End If
   gbSequenceChanged = False
End Sub

Private Sub optNucleotids_Click(Index As Integer)
Dim bShowOriginal As Boolean
   If Index = 0 Then 'Original Clicked
      bShowOriginal = True
   Else 'Optimized Clicked
      bShowOriginal = False
   End If
   Call RebuildChain(bShowOriginal)
End Sub

Name: frmStatistics.frm
Code:

VERSION 5.00
Begin VB.Form frmStatistics
   Caption = "Statistics"
   ClientHeight = 6450
   ClientLeft = 60
   ClientTop = 345
   ClientWidth = 8595
   Icon = "frmStatistics.frx":0000
   LinkTopic = "Form1 "
   MDIChild = -1 'True
   ScaleHeight = 6450
   Scale Width = 8595
   Begin VB.Frame Frame1
      Caption = "original"
      Height = 2655
      Left = 120
      TabIndex = 9
      Top = 720
      Width = 2175
      Begin VB.Label lblAdenin
         BorderStyle = 1 'Fixed Single
         Height = 495
         Left = 840
         TabIndex = 17
         Top = 240
         Width = 1215
      End
      Begin VB.Label lblCytosin
         BorderStyle = 1 'Fixed Single
         Height = 495
         Left = 840
         Tablndex = 16
         Top = 2040
         Width = 1215
      End
      Begin VB.Label lblGuanin
         BorderStyle = 1 'Fixed Single
         Height = 495
         Left = 840
         TabIndex = 15
         Top = 1440
         Width = 1215
      End
      Begin VB.Label lblThymin
         BorderStyle = 1 'Fixed Single
         Height = 495
         Left = 840
         TabIndex = 14
         Top = 840
         Width = 1215
      End
      Begin VB.Label Label14
         Caption = "Cytosin"
         Height = 255
         Left = 120
         Tablndex = 13
         Top = 2160
         Width = 615
      End
      Begin VB.Label Label2
         Caption = "Thymin"
         Height = 255
         Left = 120
         TabIndex = 12
         Top = 960
         Width = 615
      End
      Begin VB.Label Label3
         Caption = "Guanin"
         Height = 255
         Left = 120
         TabIndex = 11
         Top = 1560
         Width = 615
      End
      Begin VB.Label Label1
         Caption = "Adenin"
         Height = 255
         Left = 120
         TabIndex = 10
         Top = 360
         Width = 615
      End
   End
   Begin VB.Frame Frame2
      Caption = "optimized"
      Height = 2655
      Left = 2520
      TabIndex = 0
      Top = 720
      Width = 2175
      Begin VB.Label Label4
         Caption = "Adenin"
         Height = 255
         Left = 120
         TabIndex = 8
         Top = 360
         Width = 615
      End
      Begin VB.Label Label6
         Caption = "Cytosin"
         Height = 255
         Left = 120
         TabIndex = 7
         Top = 2160
         Width = 615
      End
      Begin VB.Label Label8
         Caption = "Guanin"
         Height = 255
         Left = 120
         TabIndex = 6
         Top = 1560
         Width = 615
      End
      Begin VB.Label Label12
         Caption = "Thymin"
         Height = 255
         Left = 120
         TabIndex = 5
         Top = 960
         Width = 615
      End
      Begin VB.Label lblOptAdenin
         BorderStyle = 1 'Fixed Single
         Height = 495
         Left = 840
         TabIndex = 4
         Top = 240
         Width = 1215
      End
      Begin VB.Label lblOptThymin
         BorderStyle = 1 'Fixed Single
         Height = 495
         Left = 840
         TabIndex = 3
         Top = 840
         Width = 1215
      End
      Begin VB.Label lblOptGuanin
         BorderStyle = 1 'Fixed Single
         Height = 495
         Left = 840
         TabIndex = 2
         Top = 1440
         Width = 1215
      End
      Begin VB.Label lblOptCytosin
         BorderStyle = 1 'Fixed Single
         Height = 495
         Left = 840
         TabIndex = 1
         Top = 2040
         Width = 1215
      End
   End
   Begin VB.Label lblBases
      BorderStyle = 1 Fixed Single
      Height = 495
      Left = 1320
      TabIndex = 19
      Top = 120
      Width = 1215
   End
   Begin VB.Label label5
      Caption = "Sum of bases"
      Height = 255
      Left = 120
      TabIndex = 18
      Top = 240
      Width = 1095
   End
End
Attribute VB_Name = "frmStatistics"
Attribute VB_GlobalNameSpace = False
Attribute VB_Creatable = False
Attribute VB_PredeclaredId = True
Attribute VB_Exposed = False
Option Explicit

Private Sub Form_Activate()
   Me.ZOrder

   lblBases.Caption = CStr(glAminoCounter * 3)
   lblAdenin.Caption = CStr(glAdenin)
   lblThymin.Caption = CStr(glThymin)
   lblGuanin.Caption = CStr(glGuanin)
   lblCytosin.Caption = CStr(glCytosin)

   Call CountBases(frmOutPut.txtOptimized.Text)
   lblOptAdenin.Caption = CStr(glOptAdenin)
   lblOptThymin.Caption = CStr(glOptThymin)
   lblOptGuanin.Caption = CStr(glOptGuanin)
   lblOptCytosin.Caption = CStr(glOptCytosin)
End Sub

Name: frmOptions.frm
Code:

VERSION 5.00
Begin VB.Form frmOptions
   Caption = "Options"
   ClientHeight = 3915
   ClientLeft = 60
   ClientTop = 345
   ClientWidth = 5760
   Icon = "frmOptions.frx":0000
   LinkTopic = "Form1"
   MDIChild = -1 'True
   ScaleHeight = 3915
   ScaleWidth = 5760
End
   Attribute VB_Name = "frmOptions"
   Attribute VB_GlobalNameSpace = False
   Attribute VB_Creatable = False
   Attribute VB_PredeclaredId = True
   Attribute VB_Exposed = False
   Option Explicit

Name: frmAbout.frm
Code:

VERSION 5.00
Begin VB.Form frmAbout
   BorderStyle = 3 'Fixed Dialog
   Caption = "About Curevac RNA-Analyzer"
   ClientHeight = 2490
   ClientLeft = 2340
   ClientTop = 1935
   ClientWidth = 6195
   ClipControls = 0 'False
   LinkTopic = "Form2"
   MaxButton = 0 'False
   MinButton = 0 'False
   ScaleHeight = 1718.642
   ScaleMode = 0 'User
   ScaleWidth = 5817.425
   ShowInTaskbar = 0 'False
   Begin VB.PictureBox picIcon
      AutoSize = -1 True
      ClipControls = 0 'False
      Height = 1215
      Left = 120
      Picture = "frmAbout.frx":0000
      ScaleHeight = 811.195
      ScaleMode = 0 'User
      ScaleWidth = 2401.98
      TabIndex = 1
      Top = 120
      Width = 3480
   End
   Begin VB.CommandButton cmdOK
      Cancel = -1 True
      Caption = "OK"
      Default = -1 True
      Height = 345
      Left = 2520
      TabIndex = 0
      Top = 2040
      Width = 1260
   End
   Begin VB.Label lblCompany
      Caption = "lblCompany"
      Height = 255
      Left = 3720
      TabIndex = 5
      Top = 1200
      Width = 2295
   End
   Begin VB.Line Line 1
      BorderColor = &H00808080&
      BorderStyle = 6 'Inside Solid
      Index = 1
      X1 = 112.686
      X2 = 5746.996
      Y1 = 1325.218
      Y2 = 1325.218
   End
   Begin VB.Label lblDescription
      Caption = "App Description"
      ForeColor = &H00000000&
      Height = 330
      Left = 120
      TabIndex = 2
      Top = 1560
      Width = 5925
   End
   Begin VB.Label lblTitle
      Caption = "Application Title"
      ForeColor = &H00000000&
      Height = 360
      Left = 3720
      TabIndex = 3
      Top = 240
      Width = 2325
   End
   Begin VB.Line Line1
      BorderColor = &H00FFFFFF&
      BorderWidth = 2
      Index = 0
      X1 = 112.686
      X2 = 5746.996
      Y1 = 1325218
      Y2 = 1325.218
   End
   Begin VB.Label lblVersion
      Caption = "Version"
      Height = 225
      Left = 3720
      TabIndex = 4
      Top = 720
      Width = 2325
   End
End
Attribute VB_Name = "frmAbout"
Attribute VB_GlobalNameSpace = False
Attribute VB_Creatable = False
Attribute VB_PredeclaredId = True
Attribute VB_Exposed = False
Option Explicit
Private Sub cmdOK_Click()
   UnloadMe
End Sub

Private Sub Form_Load()
   Me.Caption = "About "& App.Title
   lblVersion.Caption = "Version" "& App.Major &"." & App.Minor & "." & App.Revision
   lblTitle.Caption = App.Title
   lblDescription.Caption = App.FileDescription
   lblCompany.Caption = App.CompanyName
End Sub

## Patentansprüche

1. Pharmazeutische Zusammensetzung, enthaltend mindestens eine modifizierte mRNA, die für mindestens ein biologisch wirksames oder antigenes Peptid oder Polypeptid codiert, in Verbindung mit einem pharmazeutisch verträglichen Träger und/oder Vehikel, **dadurch gekennzeichnet, dass**
- der G/C-Gehalt des für das Peptid oder Polypeptid codierenden Bereichs der modifizierten mRNA größer als der G/C-Gehalt des codierenden Bereichs der für das Peptid oder Polypeptid codierenden Wildtyp-mRNA ist und die codierte Aminosäuresequenz gegenüber dem Wildtyp unverändert ist und/oder
- der für das Peptid oder Polypeptid codierende Bereich der modifizierten mRNA gegenüber dem für das Peptid oder Polypeptid codierenden Bereich der Wildtyp-mRNA derart verändert ist, dass mindestens ein Codon der Wildtyp-Sequenz, das für eine in der Zelle relativ seltene tRNA codiert, gegen ein Codon ausgetauscht ist, das für eine in der Zelle relativ häufige tRNA codiert, welche die gleiche Aminosäure trägt wie die relativ seltene tRNA.

2. Pharmazeutische Zusammensetzung nach Anspruch 1, **dadurch gekennzeichnet, dass** der G/C-Gehalt des für das Peptid oder Polypeptid codierenden Bereichs der modifizierten mRNA um mindestens 15%-Punkte größer als der G/C-Gehalt des codierenden Bereichs der für das Peptid oder Polypeptid codierenden Wildtyp-mRNA ist.

3. Pharmazeutische Zusammensetzung nach Anspruch 1 oder 2, **dadurch gekennzeichnet, dass** alle Codons der Wildtyp-Sequenz, die für eine in der Zelle relativ seltene tRNA codieren, jeweils gegen ein Codon ausgetauscht sind, das für eine in der Zelle relativ häufige tRNA codiert, welche jeweils die gleiche Aminosäure trägt wie die relativ seltene tRNA.

4. Pharmazeutische Zusammensetzung nach Anspruch 1 oder 2, **dadurch gekennzeichnet, dass** der für das Peptid oder Polypeptid codierende Bereich der modifizierten mRNA der verändert ist, dass sich ein maximaler G/C-Gehalt in Verbindung mit den Codons ergibt, die für relative häufige tRNAs codieren.

5. Pharmazeutische Zusammensetzung nach einem der Ansprüche 1 bis 4, **dadurch gekennzeichnet, dass** der für das Peptid oder Polypeptid codierende Bereich und/oder der 5'- und/oder der 3'-nicht-translatierte Bereich der modifizierten mRNA gegenüber der Wildtyp-mRNA derart verändert ist, dass er keine destabilisierenden Sequenzelemente aufweist.

6. Pharmazeutische Zusammensetzung nach einem der Ansprüche 1 bis 5, **dadurch gekennzeichnet, dass** die modifizierte mRNA eine 5'-Cap-Struktur und/oder einen polyA-Schwanz von mindestens 70 Nucleotiden und/oder eine IRES und/oder eine 5'-Stabilisierungssequenz und/oder eine 3'-Stabilisierungssequenz aufweist.

7. Pharmazeutische Zusammensetzung nach einem der Ansprüche 1 bis 6, **dadurch gekennzeichnet, dass** die modifizierte mRNA mindestens ein Anologes natürlich vorkommender Nucleotide aufweist.

8. Pharmazeutische Zusammensetzung nach Anspruch 7, **dadurch gekennzeichnet, dass** das Analoge aus der Gruppe, bestehend aus Phosphorthioaten, Phosphoramidaten, Peptidnucleotiden, Methylphosphonaten, 7-Deazaguanosin, 5-Methylcytosin und Inosin, ausgewählt ist.

9. Pharmazeutische Zusammensetzung nach einem der Ansprüche 1 bis 8, **dadurch gekennzeichnet, dass** das Polypeptid aus der Gruppe, bestehend aus Wachstumsfaktoren, Tumorantigenen, Antigenen von Viren, Bakterien und Protozoen, ausgewählt ist.

10. Pharmazeutische Zusammensetzung nach Anspruch 9, **dadurch gekennzeichnet, dass** das virale, bakterielle oder protozoologische Antigen aus einem sekretierten Protein stammt.

11. Pharmazeutische Zusammensetzung nach Anspruch 9 oder 10, **dadurch gekennzeichnet, dass** das Polypeptid ein Polyepitop von Tumorantigenen, viralen, bakteriellen oder protozoologischen Antigenen ist.

12. Pharmazeutische Zusammensetzung nach einem der Ansprüche 1 bis 11, **dadurch gekennzeichnet, dass** die mRNA zusätzlich für mindestens ein Cytokin codiert.

13. Pharmazeutische Zusammensetzung nach einem der Ansprüche 1 bis 11, welche weiter mindestens ein Cytokin enthält.

14. Pharmazeutische Zusammensetzung nach einem der Ansprüche 1 bis 13 zur Impfung gegen Infektionskrankheiten und Krebs.

15. Pharmazeutische Zusammensetzung nach einem der Ansprüche 1 bis 13 zur Geweberegeneration.
